(19) Europäisches Patentamt
European Patent Office
Office européen des brevets

(11) EP 1 264 842 A1

(12) **EUROPEAN PATENT APPLICATION**
published in accordance with Art. 158(3) EPC

(43) Date of publication:
**11.12.2002 Bulletin 2002/50**

(21) Application number: **01906155.5**

(22) Date of filing: **21.02.2001**

(51) Int Cl.[7]: **C07K 14/47**, C12N 15/12, C12N 1/21, C12N 1/19, C12N 5/10, C07K 16/18, C12Q 1/68, G01N 33/53, G01N 33/577 // C12P21:02

(86) International application number:
**PCT/JP01/01236**

(87) International publication number:
**WO 01/060859 (23.08.2001 Gazette 2001/34)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **21.02.2000 JP 2000042933**

(71) Applicant: **Kureha Chemical Industry Co., Ltd.
Tokyo 103-8552 (JP)**

(72) Inventors:
- **HIROSE, Kunitaka
Nerima-ku, Tokyo 179-0074 (JP)**
- **SAKAI, Jun
Shinjuku-ku, Tokyo 169-0073 (JP)**

(74) Representative: **Minderop, Ralph H., Dr. rer. nat.
Cohausz & Florack,
Patentanwälte,
Kanzlerstrasse 8a
40472 Düsseldorf (DE)**

# (54) NOVEL PROTEINS AND NOVEL GENES ENCODING THE SAME

(57) Novel proteins, novel genes encoding the same, plasmids respectively comprising these genes, transformants respectively comprising these plasmids, antibodies or fragments thereof against the above novel proteins, methods of detecting a bacterial infection, and novel polynucleotides are disclosed. The novel proteins are activated human macrophage-specific proteins.

EP 1 264 842 A1

Sequence Listing

<110> Kureha Chemical Industry Co., Ltd.

<120> Novel Proteins and Novel Genes Encoding the Same

<130> KRH-647

<150> JP 2000-042933
<151> 2000-02-21

<160> 10

<210> 1
<211> 2180
<212> DNA
<213> Homo sapiens

<220>
<221> CDS
<222> (37)..(1482)

<400> 1

```
ggcacgagct gaactgaacc tcttctttac aacgaa atg atg ctc aag tct atc    54
                                        Met Met Leu Lys Ser Ile
                                          1               5

aca gaa agc ttt gcc aca gca atc cat ggc ttg aaa gtg gga cac ctg   102
Thr Glu Ser Phe Ala Thr Ala Ile His Gly Leu Lys Val Gly His Leu
            10                  15                  20

aca gat cgt gtt att cag agg agc aag agg atg att cta gac act ctg   150
Thr Asp Arg Val Ile Gln Arg Ser Lys Arg Met Ile Leu Asp Thr Leu
        25                  30                  35

ggt gct ggg ttc ctg gga acc act acg gaa gtg ttt cac ata gcc agc   198
Gly Ala Gly Phe Leu Gly Thr Thr Thr Glu Val Phe His Ile Ala Ser
    40                  45                  50
```

caa tat agc aag atc tac agt tcc aac ata tcc agc act gta tgg ggt 246
Gln Tyr Ser Lys Ile Tyr Ser Ser Asn Ile Ser Ser Thr Val Trp Gly
55 60 65 70

cag cca gac atc agg ctc ccg ccc aca tat gct gct ttt gtg aac ggt 294
Gln Pro Asp Ile Arg Leu Pro Pro Thr Tyr Ala Ala Phe Val Asn Gly
75 80 85

gtg gct att cac tcc atg gat ttt gat gac acg tgg cac cct gcc acc 342
Val Ala Ile His Ser Met Asp Phe Asp Asp Thr Trp His Pro Ala Thr
90 95 100

cac cct tct ggg gct gtc ctt cct gtc ctc aca gct tta gca gaa gcc 390
His Pro Ser Gly Ala Val Leu Pro Val Leu Thr Ala Leu Ala Glu Ala
105 110 115

ctg cca agg agt cca aag ttt tct ggc ctt gac ctg ctg ctg gct ttc 438
Leu Pro Arg Ser Pro Lys Phe Ser Gly Leu Asp Leu Leu Leu Ala Phe
120 125 130

aat gtt ggt att gaa gtg caa ggc cga tta ctg cat ttc gcc aag gag 486
Asn Val Gly Ile Glu Val Gln Gly Arg Leu Leu His Phe Ala Lys Glu
135 140 145 150

gcc aat gac atg cca aag aga ttc cat ccc cct tcc gtg gta gga acg 534
Ala Asn Asp Met Pro Lys Arg Phe His Pro Pro Ser Val Val Gly Thr
155 160 165

ttg ggt agt gct gct gct gca tcc aag ttt tta gga ctt agc tcg aca 582
Leu Gly Ser Ala Ala Ala Ala Ser Lys Phe Leu Gly Leu Ser Ser Thr
170 175 180

aag tgc cga gaa gct ctg gcc att gct gtt tcc cat gct ggg gca ccc 630
Lys Cys Arg Glu Ala Leu Ala Ile Ala Val Ser His Ala Gly Ala Pro
185 190 195

atg gcc aat gct gcc acc cag acc aag ccc ctc cac att ggc aat gct 678

Met Ala Asn Ala Ala Thr Gln Thr Lys Pro Leu His Ile Gly Asn Ala
200 205 210

gcc aag cat ggg ata gaa gct gca ttt ttg gca atg ttg ggt ctc caa 726
Ala Lys His Gly Ile Glu Ala Ala Phe Leu Ala Met Leu Gly Leu Gln
215 220 225 230

gga aac aag cag gtc ttg gac ttg gag gca gga ttt ggg gcc ttt tat 774
Gly Asn Lys Gln Val Leu Asp Leu Glu Ala Gly Phe Gly Ala Phe Tyr
235 240 245

gcc aac tat tcc cca aaa gtc ctt cca agc ata gct tcc tac agt tgg 822
Ala Asn Tyr Ser Pro Lys Val Leu Pro Ser Ile Ala Ser Tyr Ser Trp
250 255 260

ctg ctg gac cag cag gac gtg gcc ttt aag cgt ttt cct gca cat tta 870
Leu Leu Asp Gln Gln Asp Val Ala Phe Lys Arg Phe Pro Ala His Leu
265 270 275

tct acc cac tgg gtg gca gac gca gct gca tct gtg aga aag cac ctt 918
Ser Thr His Trp Val Ala Asp Ala Ala Ala Ser Val Arg Lys His Leu
280 285 290

gta gca gag aga gcc ctg ctt cca act gac tac att aag aga att gtg 966
Val Ala Glu Arg Ala Leu Leu Pro Thr Asp Tyr Ile Lys Arg Ile Val
295 300 305 310

ctc agg ata cca aat gtc cag tat gta aac agg ccc ttt cca gtt tcg 1014
Leu Arg Ile Pro Asn Val Gln Tyr Val Asn Arg Pro Phe Pro Val Ser
315 320 325

gag cat gaa gcc cgt cat tca ttc cag tat gtg gcc tgt gcc atg ctg 1062
Glu His Glu Ala Arg His Ser Phe Gln Tyr Val Ala Cys Ala Met Leu
330 335 340

ctt gat ggt ggc atc act gtc ccc tca ttc cat gaa tgc cag atc aac 1110
Leu Asp Gly Gly Ile Thr Val Pro Ser Phe His Glu Cys Gln Ile Asn
345 350 355

```
agg cca cag gtg aga gag ctg ctc agt aag gtg gag ctg gag tac cct     1158
Arg Pro Gln Val Arg Glu Leu Leu Ser Lys Val Glu Leu Glu Tyr Pro
    360                 365                 370

ccg gac aac ttg cca agc ttc aac ata ctg tac tgt gaa ata agt gtc     1206
Pro Asp Asn Leu Pro Ser Phe Asn Ile Leu Tyr Cys Glu Ile Ser Val
375                 380                 385                 390

acc ctc aag gat gga gcc acc ttc aca gat cgc tct gat acc ttc tat     1254
Thr Leu Lys Asp Gly Ala Thr Phe Thr Asp Arg Ser Asp Thr Phe Tyr
                395                 400                 405

ggg cac tgg aga aaa cca ctg agc cag gag gac cta gag gaa aag ttc     1302
Gly His Trp Arg Lys Pro Leu Ser Gln Glu Asp Leu Glu Glu Lys Phe
            410                 415                 420

aga gcc aat gcc tcc aag atg ctg tcc tgg gac aca gtg gaa agc ctt     1350
Arg Ala Asn Ala Ser Lys Met Leu Ser Trp Asp Thr Val Glu Ser Leu
        425                 430                 435

ata aag ata gtc aaa aat cta gaa gac cta gaa gac tgt tct gtg tta     1398
Ile Lys Ile Val Lys Asn Leu Glu Asp Leu Glu Asp Cys Ser Val Leu
    440                 445                 450

act aca ctt ctc aaa gga ccc tct cca cca gag gta gct tca aac tct     1446
Thr Thr Leu Leu Lys Gly Pro Ser Pro Pro Glu Val Ala Ser Asn Ser
455                 460                 465                 470

cca gca tgt aat aat tct atc aca aat ctc tcc tgaggcttac caacatctaa 1499
Pro Ala Cys Asn Asn Ser Ile Thr Asn Leu Ser
                475                 480

atgactttgc atttggggag attcaatgat ttggtttgta aagcaagggt ctgctgcttg 1559

gttttcccag gaaaaatgaa caaagatgga gagagtccag aaacagaact acatatatct 1619

ggaaggagcc ttctcctgaa aattttgcag gacagttcca cttacctaaa tcaagatgaa 1679
```

acacacacac aaaaatgagt ttgtaagcat tcacaagggt gaaattcaac tcacctgtga 1739

tttacttata aaattaatct cttcatagga attatgtgtg gacttcatga gcctcaaggt 1799

tttagaggga tgtgaacctg catgtatatt ttctgacagt ggagagggct ctggtgcatt 1859

gtgtcaccaa cagatctcct agaccatggc ttattaccaa gccctccaca gtgcaagggg 1919

tgctactggg gaatgggtgg gtttaaatcc tgcctctgcc attcactaga tgtagccttg 1979

agcatgttac cattagccct ctgcctcagt ttccctattt gtcaagccga agtaaaaagc 2039

agtctggaaa aatcgcattt tggctctaga acccatggtc ttaagcactg caatatatca 2099

cctttcagta taaaaatatt tgaatcagag ttgcaataaa gaatgaaaag gaaaaaagag 2159

aagtaaaaaa aaaaaaaaaa a 2180

<210> 2
<211> 481
<212> PRT
<213> Homo sapiens

<400> 2
Met Met Leu Lys Ser Ile Thr Glu Ser Phe Ala Thr Ala Ile His Gly
1 5 10 15

Leu Lys Val Gly His Leu Thr Asp Arg Val Ile Gln Arg Ser Lys Arg
20 25 30

Met Ile Leu Asp Thr Leu Gly Ala Gly Phe Leu Gly Thr Thr Thr Glu
35 40 45

Val Phe His Ile Ala Ser Gln Tyr Ser Lys Ile Tyr Ser Ser Asn Ile
50 55 60

Ser Ser Thr Val Trp Gly Gln Pro Asp Ile Arg Leu Pro Pro Thr Tyr
65 70 75 80

Ala Ala Phe Val Asn Gly Val Ala Ile His Ser Met Asp Phe Asp Asp
85 90 95

Thr Trp His Pro Ala Thr His Pro Ser Gly Ala Val Leu Pro Val Leu
100 105 110

Thr Ala Leu Ala Glu Ala Leu Pro Arg Ser Pro Lys Phe Ser Gly Leu
115 120 125

Asp Leu Leu Leu Ala Phe Asn Val Gly Ile Glu Val Gln Gly Arg Leu
130 135 140

Leu His Phe Ala Lys Glu Ala Asn Asp Met Pro Lys Arg Phe His Pro
145 150 155 160

Pro Ser Val Val Gly Thr Leu Gly Ser Ala Ala Ala Ala Ser Lys Phe
165 170 175

Leu Gly Leu Ser Ser Thr Lys Cys Arg Glu Ala Leu Ala Ile Ala Val
180 185 190

Ser His Ala Gly Ala Pro Met Ala Asn Ala Ala Thr Gln Thr Lys Pro
195 200 205

Leu His Ile Gly Asn Ala Ala Lys His Gly Ile Glu Ala Ala Phe Leu
210 215 220

Ala Met Leu Gly Leu Gln Gly Asn Lys Gln Val Leu Asp Leu Glu Ala
225 230 235 240

Gly Phe Gly Ala Phe Tyr Ala Asn Tyr Ser Pro Lys Val Leu Pro Ser
245 250 255

Ile Ala Ser Tyr Ser Trp Leu Leu Asp Gln Gln Asp Val Ala Phe Lys
260 265 270

Arg Phe Pro Ala His Leu Ser Thr His Trp Val Ala Asp Ala Ala Ala
275 280 285

Ser Val Arg Lys His Leu Val Ala Glu Arg Ala Leu Leu Pro Thr Asp
290 295 300

Tyr Ile Lys Arg Ile Val Leu Arg Ile Pro Asn Val Gln Tyr Val Asn
305 310 315 320

Arg Pro Phe Pro Val Ser Glu His Glu Ala Arg His Ser Phe Gln Tyr
325 330 335

Val Ala Cys Ala Met Leu Leu Asp Gly Gly Ile Thr Val Pro Ser Phe
340 345 350

His Glu Cys Gln Ile Asn Arg Pro Gln Val Arg Glu Leu Leu Ser Lys
355 360 365

Val Glu Leu Glu Tyr Pro Pro Asp Asn Leu Pro Ser Phe Asn Ile Leu
370 375 380

Tyr Cys Glu Ile Ser Val Thr Leu Lys Asp Gly Ala Thr Phe Thr Asp
385 390 395 400

Arg Ser Asp Thr Phe Tyr Gly His Trp Arg Lys Pro Leu Ser Gln Glu
405 410 415

Asp Leu Glu Glu Lys Phe Arg Ala Asn Ala Ser Lys Met Leu Ser Trp
420 425 430

Asp Thr Val Glu Ser Leu Ile Lys Ile Val Lys Asn Leu Glu Asp Leu
435 440 445

Glu Asp Cys Ser Val Leu Thr Thr Leu Leu Lys Gly Pro Ser Pro Pro
450 455 460

Glu Val Ala Ser Asn Ser Pro Ala Cys Asn Asn Ser Ile Thr Asn Leu

465 470 475 480

Ser

<210> 3
<211> 1970
<212> DNA
<213> Homo sapiens

<220>
<221> CDS
<222> (126)..(1298)

<400> 3
ggcacgaggc cagccaatat agcaagatct acagttccaa catatccagc actgtttggg 60

gtcagccaga catcaggctc ccgcccacat atgctgcttt tgtgaacggt gtggctattc 120

actcc atg gat ttt gat gac acg tgg cac cct gcc acc cac cct tct ggg 170
Met Asp Phe Asp Asp Thr Trp His Pro Ala Thr His Pro Ser Gly
1 5 10 15

gct gtc ctt cct gtc ctc aca gct tta gca gaa gcc ctg cca agg agt 218
Ala Val Leu Pro Val Leu Thr Ala Leu Ala Glu Ala Leu Pro Arg Ser
20 25 30

cca aag ttt tct ggc ctt gac ctg ctg ctg gct ttc aat gtt ggt att 266
Pro Lys Phe Ser Gly Leu Asp Leu Leu Leu Ala Phe Asn Val Gly Ile
35 40 45

gaa gtg caa ggc cga tta ctg cat ttc gcc aag gag gcc aat gac atg 314
Glu Val Gln Gly Arg Leu Leu His Phe Ala Lys Glu Ala Asn Asp Met
50 55 60

cca aag aga ttc cat ccc cct tcc gtg gta gga acg ttg ggt agt gct 362
Pro Lys Arg Phe His Pro Pro Ser Val Val Gly Thr Leu Gly Ser Ala
65 70 75

gct gct gca tcc aag ttt tta gga ctt agc tcg aca aag tgc cga gaa 410
Ala Ala Ala Ser Lys Phe Leu Gly Leu Ser Ser Thr Lys Cys Arg Glu
80 85 90 95

gct ctg gcc att gct gtt tcc cat gct ggg gca ccc atg gcc aat gct 458
Ala Leu Ala Ile Ala Val Ser His Ala Gly Ala Pro Met Ala Asn Ala
100 105 110

gcc acc cag acc aag ccc ctc cac att ggc aat gct gcc aag cat ggg 506
Ala Thr Gln Thr Lys Pro Leu His Ile Gly Asn Ala Ala Lys His Gly
115 120 125

ata gaa gct gca ttt ttg gca atg ttg ggt ctc caa gga aac aag cag 554
Ile Glu Ala Ala Phe Leu Ala Met Leu Gly Leu Gln Gly Asn Lys Gln
130 135 140

gtc ttg gac ttg gag gca gga ttt ggg gcc ttt tat gcc aac tat tcc 602
Val Leu Asp Leu Glu Ala Gly Phe Gly Ala Phe Tyr Ala Asn Tyr Ser
145 150 155

cca aaa gtc ctt cca agc ata gct tcc tac agt tgg ctg ctg gac cag 650
Pro Lys Val Leu Pro Ser Ile Ala Ser Tyr Ser Trp Leu Leu Asp Gln
160 165 170 175

cag gac gtg gcc ttt aag cgt ttt cct gca cat tta tct acc cac tgg 698
Gln Asp Val Ala Phe Lys Arg Phe Pro Ala His Leu Ser Thr His Trp
180 185 190

gtg gca gac gca gct gca tct gtg aga aag cac ctt gta gca gag aga 746
Val Ala Asp Ala Ala Ala Ser Val Arg Lys His Leu Val Ala Glu Arg
195 200 205

gcc ctg ctt cca act gac tac att aag aga att gtg ctc agg ata cca 794
Ala Leu Leu Pro Thr Asp Tyr Ile Lys Arg Ile Val Leu Arg Ile Pro
210 215 220

aat gtc cag tat gta aac agg ccc ttt cca gtt tcg gag cat gaa gcc 842

```
Asn Val Gln Tyr Val Asn Arg Pro Phe Pro Val Ser Glu His Glu Ala
    225                 230                 235

cgt cat tca ttc cag tat gtg gcc tgt gcc atg ctg ctt gat ggt ggc      890
Arg His Ser Phe Gln Tyr Val Ala Cys Ala Met Leu Leu Asp Gly Gly
240                 245                 250                 255

atc act gtc ccc tca ttc cat gaa tgc cag atc aac agg cca cag gtg      938
Ile Thr Val Pro Ser Phe His Glu Cys Gln Ile Asn Arg Pro Gln Val
                260                 265                 270

aga gag ctg ctc agt aag gtg gag ctg gag tac cct ccg gac aac ttg      986
Arg Glu Leu Leu Ser Lys Val Glu Leu Glu Tyr Pro Pro Asp Asn Leu
            275                 280                 285

cca agc ttc aac ata ctg tac tgt gaa ata agt gtc acc ctc aag gat      1034
Pro Ser Phe Asn Ile Leu Tyr Cys Glu Ile Ser Val Thr Leu Lys Asp
        290                 295                 300

gga gcc acc ttc aca gat cgc tct gat acc ttc tat ggg cac tgg aga      1082
Gly Ala Thr Phe Thr Asp Arg Ser Asp Thr Phe Tyr Gly His Trp Arg
    305                 310                 315

aaa cca ctg agc cag gag gac cta gag gaa aag ttc aga gcc aat gcc      1130
Lys Pro Leu Ser Gln Glu Asp Leu Glu Glu Lys Phe Arg Ala Asn Ala
320                 325                 330                 335

tcc aag atg ctg tcc tgg gac aca gtg gaa agc ctt ata aag ata gtc      1178
Ser Lys Met Leu Ser Trp Asp Thr Val Glu Ser Leu Ile Lys Ile Val
                340                 345                 350

aaa aat cta gaa gac cta gaa gac tgt tct gtg tta act aca ctt ctc      1226
Lys Asn Leu Glu Asp Leu Glu Asp Cys Ser Val Leu Thr Thr Leu Leu
            355                 360                 365

aaa gga ccc tct cca cca gag gta gct tca aac tct cca gca tgt aat      1274
Lys Gly Pro Ser Pro Pro Glu Val Ala Ser Asn Ser Pro Ala Cys Asn
        370                 375                 380
```

aat tct atc aca aat ctc tcc tgaggcttac caacatctaa atgactttgc 1325
Asn Ser Ile Thr Asn Leu Ser
385 390

atttggggag attcaatgat ttggtttgta aagcaagggt ctgctgcttg gttttcccag 1385

gaaaaatgaa caaagatgga gagagtccag aaacagaact acatatatct ggaaggagcc 1445

ttctcctgaa aattttgcag gacagttcca cttacctaaa tcaagatgaa acacacacac 1505

aaaaatgagt ttgtaagcat tcacaagggt gaaattcaac tcacctgtga tttacttata 1565

aaattaatct cttcatagga attatgtgtg gacttcatga gcctcaaggt tttagaggga 1625

tgtgaacctg catgtatatt ttctgacagt ggagagggct ctggtgcatt gtgtcaccaa 1685

cagatctcct agaccatggc ttattaccaa gccctccaca gtgcaagggg tgctactggg 1745

gaatgggtgg gtttaaatcc tgcctctgcc attcactaga tgtagccttg agcatgttac 1805

cattagccct ctgcctcagt ttccctattt gtcaagccga agtaaaaagc agtctggaaa 1865

aatcgcattt tggctctaga acccatggtc ttaagcactg caatatatca cctttcagta 1925

taaaaatatt tgaatcagag ttgcaataaa aaaaaaaaaa aaaaa 1970

<210> 4
<211> 390
<212> PRT
<213> Homo sapiens

<400> 4
Met Asp Phe Asp Asp Thr Trp His Pro Ala Thr His Pro Ser Gly Ala
1 5 10 15

Val Leu Pro Val Leu Thr Ala Leu Ala Glu Ala Leu Pro Arg Ser Pro

20 25 30

Lys Phe Ser Gly Leu Asp Leu Leu Leu Ala Phe Asn Val Gly Ile Glu
35 40 45

Val Gln Gly Arg Leu Leu His Phe Ala Lys Glu Ala Asn Asp Met Pro
50 55 60

Lys Arg Phe His Pro Pro Ser Val Val Gly Thr Leu Gly Ser Ala Ala
65 70 75 80

Ala Ala Ser Lys Phe Leu Gly Leu Ser Ser Thr Lys Cys Arg Glu Ala
85 90 95

Leu Ala Ile Ala Val Ser His Ala Gly Ala Pro Met Ala Asn Ala Ala
100 105 110

Thr Gln Thr Lys Pro Leu His Ile Gly Asn Ala Ala Lys His Gly Ile
115 120 125

Glu Ala Ala Phe Leu Ala Met Leu Gly Leu Gln Gly Asn Lys Gln Val
130 135 140

Leu Asp Leu Glu Ala Gly Phe Gly Ala Phe Tyr Ala Asn Tyr Ser Pro
145 150 155 160

Lys Val Leu Pro Ser Ile Ala Ser Tyr Ser Trp Leu Leu Asp Gln Gln
165 170 175

Asp Val Ala Phe Lys Arg Phe Pro Ala His Leu Ser Thr His Trp Val
180 185 190

Ala Asp Ala Ala Ala Ser Val Arg Lys His Leu Val Ala Glu Arg Ala
195 200 205

Leu Leu Pro Thr Asp Tyr Ile Lys Arg Ile Val Leu Arg Ile Pro Asn
210 215 220

Val Gln Tyr Val Asn Arg Pro Phe Pro Val Ser Glu His Glu Ala Arg
225 230 235 240

His Ser Phe Gln Tyr Val Ala Cys Ala Met Leu Leu Asp Gly Gly Ile
245 250 255

Thr Val Pro Ser Phe His Glu Cys Gln Ile Asn Arg Pro Gln Val Arg
260 265 270

Glu Leu Leu Ser Lys Val Glu Leu Glu Tyr Pro Pro Asp Asn Leu Pro
275 280 285

Ser Phe Asn Ile Leu Tyr Cys Glu Ile Ser Val Thr Leu Lys Asp Gly
290 295 300

Ala Thr Phe Thr Asp Arg Ser Asp Thr Phe Tyr Gly His Trp Arg Lys
305 310 315 320

Pro Leu Ser Gln Glu Asp Leu Glu Glu Lys Phe Arg Ala Asn Ala Ser
325 330 335

Lys Met Leu Ser Trp Asp Thr Val Glu Ser Leu Ile Lys Ile Val Lys
340 345 350

Asn Leu Glu Asp Leu Glu Asp Cys Ser Val Leu Thr Thr Leu Leu Lys
355 360 365

Gly Pro Ser Pro Pro Glu Val Ala Ser Asn Ser Pro Ala Cys Asn Asn
370 375 380

Ser Ile Thr Asn Leu Ser
385 390

<210> 5
<211> 652
<212> DNA
<213> Homo sapiens

<220>
<221> CDS
<222> (56)..(307)

<400> 5
ggcaccaggc gcaccgcccg gcgtccagat ttggcaattc ttcgctgaag tcatc atg 58
Met
1

agc ttt ttc caa ctc ctg atg aaa agg aag gaa ctc att ccc ttg gtg 106
Ser Phe Phe Gln Leu Leu Met Lys Arg Lys Glu Leu Ile Pro Leu Val
5 10 15

gtg ttc atg act gtg gcg gcg ggt gga gcc tca tct ttc gct gtg tat 154
Val Phe Met Thr Val Ala Ala Gly Gly Ala Ser Ser Phe Ala Val Tyr
20 25 30

tct ctt tgg aaa acc gat gtg atc ctt gat cga aaa aaa aat cca gaa 202
Ser Leu Trp Lys Thr Asp Val Ile Leu Asp Arg Lys Lys Asn Pro Glu
35 40 45

cct tgg gaa act gtg gac cct act gta cct caa aag ctt ata aca atc 250
Pro Trp Glu Thr Val Asp Pro Thr Val Pro Gln Lys Leu Ile Thr Ile
50 55 60 65

aac caa caa tgg aaa ccc att gaa gag ttg caa aat gtc caa agg gtg 298
Asn Gln Gln Trp Lys Pro Ile Glu Glu Leu Gln Asn Val Gln Arg Val
70 75 80

acc aaa tgacgagccc tcgcctcttt cttctgaaga gtactctata aatctagtgg 354
Thr Lys

aaacatttct gcacaaacta gattctggac accagtgtgc ggaaatgctt ctgctacatt 414

tttagggttt gtctacattt tttgggctct ggataaggaa ttaaaggagt gcagcaataa 474

ctgcactgtc taaaagtttg tgcttatttt cttgtaaatt tgaatattgc atattgaaat 534

ttttgtttat gatctatgaa tgtttttctt aaaatttaca aagctttgta aattagattt 594

tctttaataa aatgccattt gtgcaagatt tctcaaagaa aaaaaaaaaa aaaaaaa 652

<210> 6
<211> 83
<212> PRT
<213> Homo sapiens

<400> 6
Met Ser Phe Phe Gln Leu Leu Met Lys Arg Lys Glu Leu Ile Pro Leu
1 5 10 15

Val Val Phe Met Thr Val Ala Ala Gly Gly Ala Ser Ser Phe Ala Val
20 25 30

Tyr Ser Leu Trp Lys Thr Asp Val Ile Leu Asp Arg Lys Lys Asn Pro
35 40 45

Glu Pro Trp Glu Thr Val Asp Pro Thr Val Pro Gln Lys Leu Ile Thr
50 55 60

Ile Asn Gln Gln Trp Lys Pro Ile Glu Glu Leu Gln Asn Val Gln Arg
65 70 75 80

Val Thr Lys

<210> 7
<211> 34
<212> DNA
<213> Artificial Sequence

<220>
<223> Description of Artificial Sequence: NLG-2 forward
primer

<400> 7
cacggatcca ttcttcgctg aagtcatcat gagc 34

<210> 8
<211> 34
<212> DNA
<213> Artificial Sequence

<220>
<223> Description of Artificial Sequence: NLG-2 reverse primer

<400> 8
gtggaattct ttggtcaccc tttggacatt ttgc 34

<210> 9
<211> 35
<212> DNA
<213> Artificial Sequence

<220>
<223> Description of Artificial Sequence: NLG-1-1 forward primer

<400> 9
cacggatcct tctttacaac gaaatgatgc tcaag 35

<210> 10
<211> 39
<212> DNA
<213> Artificial Sequence

<220>
<223> Description of Artificial Sequence: NLG-1-1 reverse

primer

<400> 10
gtggaattcg gagagatttg tgatagaatt attacatgc 39

## Description

TECHNICAL FIELD

The present invention relates to plural novel proteins, a novel gene encoding each of the proteins, a plasmid comprising each of the genes, a transformant comprising each of the plasmids, an antibody or a fragment thereof against each of the novel proteins, a method for detecting a bacterial infection, and novel polynucleotides. The novel proteins of the present invention are activated human macrophage-specific proteins.

BACKGROUND ART

It is known that a Lipopolysaccharide (LPS) is a glycolipid existing in an outer membrane of Gram-negative bacterium and activates a macrophage to induce an expression of many genes. Known examples of such genes having expressions that are induced by the LPS are those of interleukin (IL)-1, exhibiting an antitumor function or an inflammatory function, such as a function to cause an inflammation by a bacterial infection, IL-6, IL-12, IL-15, IL-18, a tumor necrosis factor (TNF), or a chemokine (such as IL-8 or MCP); granulocyte colony-stimulating factor (G-CSF) exhibiting a hemapoietic function, monocyte (M)-CSF, or GM-CSF; or collagenase playing a main role in an inflammation such as that caused by a bacterial infection, cyclooxygenase (COX), or a nitrogen oxide synthase (iNOS) or the like. Almost all of the above proteins encoded by the above genes are physiologically active proteins playing important roles in a body [Annu. Rev. Immunol., 2, 283-318 (1984); Inflammation: Basic Principles and Clinical Correlates, 637-662, Raven Press Ltd., New York (1992)].

There are about 0.1 million genes in the human chromosome, but only 10 or 20 percentage thereof have been isolated and identified. Therefore, as almost all of the genes have not been isolated or analyzed, it is believed that almost all of the genes having an expression that is specifically induced by LPS are unidentified novel genes.

Septicemia is a systemic disease wherein a festering lesion exists in a body, and many bacteria are intermittently or continuously introduced into the blood from the festering lesion. A diagnosis of the septicemia is carried out by culturing the blood, and when the existence of bacteria is proved, the illness is definitely diagnosed as the septicemia. However, the above method has disadvantages, namely, the method is time-consuming, and when a blood sample is drawn, it may be contaminated with bacteria from skin, such as *Staphylococcus epidermidis*.

Therefore, the inventors of the present invention made an intensive search for genes having an expression that is induced specifically at a macrophage by an LPS-stimulation, for the purpose of an application for developing a new method of diagnosis and/or medicament for treating a disease such as inflammation, allergy, or cancer, particularly a bacterial infection. As a result, three novel genes were isolated and identified. Further, the present inventors found that these three genes were not expressed in healthy persons, but were expressed in patients suffering from a bacterial infection. The invention is based on the above findings.

DISCLOSURE OF INVENTION

The present invention relates to

(1) a protein comprising an amino acid sequence of SEQ ID NO: 2 in the sequence listing, or a variation functionally equivalent thereto, or a fragment of the protein or the variation (hereinafter sometimes collectively referred to as a "first novel protein of the present invention"),
(2) a protein comprising an amino acid sequence of SEQ ID NO: 4 in the sequence listing, or a variation functionally equivalent thereto, or a fragment of the protein or the variation (hereinafter sometimes collectively referred to as a "second novel protein of the present invention"), and
(3) a protein comprising an amino acid sequence of SEQ ID NO: 6 in the sequence listing, or a variation functionally equivalent thereto, or a fragment of the protein or the variation (hereinafter sometimes collectively referred to as a "third novel protein of the present invention").

Further, the present invention relates to

(1) a gene encoding the above-mentioned "first novel protein of the present invention" (hereinafter sometimes referred to as a "first novel gene of the present invention"),
(2) a gene encoding the above-mentioned "second novel protein of the present invention" (hereinafter sometimes referred to as a "second novel gene of the present invention"), and
(3) a gene encoding the above-mentioned "third novel protein of the present invention" (hereinafter sometimes referred to as a "third novel gene of the present invention").

Further, the present invention relates to plasmids comprising each of the above-mentioned genes.
Further, the present invention relates to transformants comprising each of the above-mentioned plasmids.
Further, the present invention relates to antibodies or fragments thereof, characterized by being reactive specifically to each of the above-mentioned proteins or variations functionally equivalent thereto.
Further, the present invention relates to a method for detecting a bacterial infection, characterized by analyzing the proteins or the variations functionally equivalent thereto, or the mRNAs thereof, in a sample to be detected.
Further, the present invention relates to

(1) a polynucleotide capable of specifically hybridizing to an mRNA consisting of an base sequence of SEQ ID NO: 1 in the sequence listing (hereinafter sometimes referred to as a "first probe of the present invention"),
(2) a polynucleotide capable of specifically hybridizing to an mRNA consisting of an base sequence of SEQ ID NO: 3 in the sequence listing (hereinafter sometimes referred to as a "second probe of the present invention"), and
(3) a polynucleotide capable of specifically hybridizing to an mRNA consisting of an base sequence of SEQ ID NO: 5 in the sequence listing (hereinafter sometimes referred to as a "third probe of the present invention").

The term "variation functionally equivalent" as used herein means a protein having an amino acid sequence wherein one or more (particularly one or several) amino acids are deleted in, changed in, or added to the amino acid sequence of an original protein, and exhibiting the same activities as the original protein. The term "added" as used herein includes an addition of one or more (particularly one or several) amino acids to an N-terminus and/or a C-terminus of an amino acid sequence, and an insertion of one or more (particularly one or several) amino acids to an inside of an amino acid sequence.
Further, the term "homologous protein" as used herein means a protein comprising an amino acid sequence having a 90% or more (preferably 95% or more, more preferably 98% or more, most preferably 99% or more) homology with the amino acid sequence of an original protein, and exhibiting the same activities as the original protein. The term "homology" as used herein means a value calculated by BLAST [Basic local alignment search tool; Altschul, S. F. et al., J. Mol. Biol., 215, 403-410, (1990)].
Furthermore, the terms "gene" and "polynucleotide" as used herein include both of DNA and RNA.

BRIEF DESCRIPTION OF DRAWINGS

Fig. 1 shows the results of electrophoresis wherein the expression of three novel genes of the present invention in human macrophages stimulated by LPS or not stimulated by LPS was detected by a northern blotting method.
Fig. 2 shows the results of electrophoresis wherein the tissue-specific expression of three novel genes of the present invention was detected by a northern blotting method.
Fig. 3 provides micrographs showing the results of the expression of the gene NLG-1-1 of the present invention in COS-1 cells.
Fig. 4 provides micrographs showing the results of the expression of the gene NLG-2 of the present invention in COS-1 cells.
Fig. 5 provides micrographs showing the results of a FISH analysis of the gene NLG-1-1 of the present invention.
Fig. 6 provides photographs showing the results of electrophoresis wherein the expression of three novel genes of the present invention in healthy persons and patients suffering from septicemia was detected by a northern blotting method.

BEST MODE FOR CARRYING OUT THE INVENTION

The present invention will be explained in detail hereinafter.
The first novel protein of the present invention includes

(1) a protein comprising an amino acid sequence of SEQ ID NO: 2 in the sequence listing,
(2) a variation functionally equivalent to the protein (1),
(3) a protein homologous to the protein (1), and
(4) a fragment thereof [i.e., a fragment of the protein (1), the variation (2), or the homologous protein (3)]. A protein consisting of the amino acid sequence of SEQ ID NO: 2 in the sequence listing, or a variation functionally equivalent or protein homologous thereto are preferred.

The protein consisting of the amino acid sequence of SEQ ID NO: 2 in the sequence listing consists of 481 amino acid residues. The protein consisting of the amino acid sequence of SEQ ID NO: 2 in the sequence listing has a high homology of approximately 82% in the amino acid sequence with mouse IRG-1 (1mmune-responsive protein-1) [Immnogenetics, 41, 263-270, (1995)], and thus seems to be human IRG-1.

In the amino acid sequence of SEQ ID NO: 2 in the sequence listing, there exist eight known sites to be phospholylated by protein kinase C and ten known sites to be phospholylated by casein kinase C. As a result, it is believed that the protein consisting of the amino acid sequence of SEQ ID NO: 2 in the sequence listing plays an important role in an intracellular signal transduction system which transduces information of LPS-stimulation.

Further, a signal peptide sequence at the N-terminus does not exist, and thus, it is believed that the protein consisting of the amino acid sequence of SEQ ID NO: 2 in the sequence listing exhibits biological activities in cells.

As the protein comprising the amino acid sequence of SEQ ID NO: 2 in the sequence listing, there may be mentioned, for example, a fusion protein of the protein consisting of the amino acid sequence of SEQ ID NO: 2 in the sequence listing with a fusion partner. In the fusion protein, the fusion partner may be linked with the N-terminus and/or C terminus of the protein consisting of the amino acid sequence of SEQ ID NO: 2.

As the fusion partner, for example, a protein for purification such as the whole or a part of glutathione-S-transferase (GST), a protein for detection such as the whole or a part of β-galactosidase α peptide (LacZ α), or a protein for expression such as a signal sequence may be used.

Further, in the fusion protein, an amino acid sequence which may be restrictively digested with a proteolytic enzyme such as thrombin or factor Xa may be optionally inserted between the protein consisting of the amino acid sequence of SEQ ID NO: 2 in the sequence listing and the fusion partner.

The fragment of the protein comprising the amino acid sequence of SEQ ID NO: 2 in the sequence listing or the variation functionally equivalent or protein homologous thereto is not particularly limited, so long as it may be used as an immunogen to prepare the first antibody or fragment thereof according to the present invention, but preferably consists of 13 or more amino acid residues, more preferably 20 or more amino acid residues, most preferably 50 or more amino acid residues.

The first novel protein of the present invention may be obtained by various known methods. For example, the protein may be prepared by using a known genetic engineering technique and the first novel gene of the present invention.

The first novel gene of the present invention is not particularly limited, so long as it encodes the first novel protein of the present invention. As the gene, there may be mentioned, for example, a gene consisting of the 37th to 1479th bases in the base sequence of SEQ ID NO: 1 in the sequence listing.

The gene consisting of the 37th to 1479th bases in the base sequence of SEQ ID NO: 1 in the sequence listing encodes the protein consisting of the amino acid sequence of SEQ ID NO: 2 in the sequence listing. Further, the gene consisting of the 37th to 1479th bases in the base sequence of SEQ ID NO: 1 in the sequence listing is not expressed in healthy persons, but is expressed in patients suffering from a bacterial infection.

The first probe of the present invention is not particularly limited, so long as it is capable of specifically hybridizing to an mRNA consisting of the base sequence of SEQ ID NO: 1 in the sequence listing. As the probe, there may be mentioned, for example, a single or double stranded polynucleotide consisting of a base sequence complementary to that of SEQ ID NO: 1 in the sequence listing, or a partial base sequence thereof. The lower limit of the number of bases in the first probe of the present invention is not particularly limited, but is preferably 18 or more, more particularly 26 or more, most particularly 41 or more. Further, the upper limit thereof is not particularly limited, but is preferably 2180 or less. The expression "specifically hybridize with an mRNA consisting of the base sequence of SEQ ID NO: 1 in the sequence listing" as used herein means that a polynucleotide does not hybridize with mRNAs derived from a healthy person, but will hybridize with the mRNA consisting of the base sequence of SEQ ID NO: 1 in the sequence listing, under the conditions described in Example 1(4). In those conditions, it is twice washed with 2xSSC (standard sodium citrate) containing 0.1% sodium dodecyl sulfate (SDS) at room temperature for 20 minutes, and further twice washed with 0.2xSSC containing 0.1% SDS at 65°C for 20 minutes.

The second novel protein of the present invention includes

(1) a protein comprising an amino acid sequence of SEQ ID NO: 4 in the sequence listing,
(2) a variation functionally equivalent to the protein (1),
(3) a protein homologous to the protein (1), and
(4) a fragment thereof [i.e., a fragment of the protein (1), the variation (2), or the homologous protein (3)]. A protein consisting of the amino acid sequence of SEQ ID NO: 4 in the sequence listing, or a variation functionally equivalent or protein homologous thereto are preferred.

The protein consisting of the amino acid sequence of SEQ ID NO: 4 in the sequence listing consists of 390 amino acid residues. The protein consisting of the amino acid sequence of SEQ ID NO: 4 in the sequence listing has a high homology of approximately 82% in the amino acid sequence with mouse IRG-1 (Immune-responsive protein-1)

[Immnogenetics, 41, 263-270, (1995)], and thus seems to be human IRG-1.

In the amino acid sequence of SEQ ID NO: 4 in the sequence listing, there exist six known sites to be phospholylated by protein kinase C and eight known sites to be phospholylated by casein kinase C. As a result, it is believed that the protein consisting of the amino acid sequence of SEQ ID NO: 4 in the sequence listing plays an important role in an intracellular signal transduction system which transduces information of LPS-stimulation.

Further, a signal peptide sequence at the N-terminus does not exist, and thus, it is believed that the protein consisting of the amino acid sequence of SEQ ID NO: 4 in the sequence listing exhibits biological activities in cells.

As the protein comprising the amino acid sequence of SEQ ID NO: 4 in the sequence listing, there may be mentioned, for example, a fusion protein of the protein consisting of the amino acid sequence of SEQ ID NO: 4 in the sequence listing with a fusion partner. In the fusion protein, the fusion partner may be linked with the N-terminus and/or C terminus of the protein consisting of the amino acid sequence of SEQ ID NO: 4.

As the fusion partner, for example, a protein for purification such as the whole or a part of glutathione-S-transferase (GST), a protein for detection such as the whole or a part of β-galactosidase α peptide (LacZ α), or a protein for expression such as a signal sequence may be used.

Further, in the fusion protein, an amino acid sequence which may be restrictively digested with a proteolytic enzyme such as thrombin or factor Xa may be optionally inserted between the protein consisting of the amino acid sequence of SEQ ID NO: 4 in the sequence listing and the fusion partner.

The fragment of the protein comprising the amino acid sequence of SEQ ID NO: 4 in the sequence listing or the variation functionally equivalent or protein homologous thereto is not particularly limited, so long as it may be used as an immunogen to prepare the second antibody or fragment thereof according to the present invention, but preferably consists of 13 or more amino acid residues, more preferably 20 or more amino acid residues, most preferably 50 or more amino acid residues.

The second novel protein of the present invention may be obtained by various known methods. For example, the protein may be prepared by using a known genetic engineering technique and the second novel gene of the present invention.

The second novel gene of the present invention is not particularly limited, so long as it encodes the second novel protein of the present invention. As the gene, there may be mentioned, for example, a gene consisting of the 126th to 1295th bases in the base sequence of SEQ ID NO: 3 in the sequence listing.

The gene consisting of the 126th to 1295th bases in the base sequence of SEQ ID NO: 3 in the sequence listing encodes the protein consisting of the amino acid sequence of SEQ ID NO: 4 in the sequence listing. Further, the gene consisting of the 126th to 1295th bases in the base sequence of SEQ ID NO: 3 in the sequence listing is not expressed in healthy persons, but is expressed in patients suffering from a bacterial infection.

The second probe of the present invention is not particularly limited, so long as it is capable of specifically hybridizing to an mRNA consisting of the base sequence of SEQ ID NO: 3 in the sequence listing. As the probe, there may be mentioned, for example, a single or double stranded polynucleotide consisting of a base sequence complementary to that of SEQ ID NO: 3 in the sequence listing, or a partial base sequence thereof. The lower limit of the number of bases in the second probe of the present invention is not particularly limited, but is preferably 18 or more, more particularly 26 or more, most particularly 41 or more. Further, the upper limit thereof is not particularly limited, but is preferably 1970 or less. The expression "specifically hybridize with an mRNA consisting of the base sequence of SEQ ID NO: 3 in the sequence listing" as used herein means that a polynucleotide does not hybridize with mRNAs derived from a healthy person, but will hybridize with the mRNA consisting of the base sequence of SEQ ID NO: 3 in the sequence listing, under the conditions described in Example 1(4).

The third novel protein of the present invention includes
a protein comprising an amino acid sequence of SEQ ID NO: 6 in the sequence listing,
(2) a variation functionally equivalent to the protein (1), (3) a protein homologous to the protein (1), and
(4) a fragment thereof [i.e., a fragment of the protein (1), the variation (2), or the homologous protein (3)]. A protein consisting of the amino acid sequence of SEQ ID NO: 6 in the sequence listing, or a variation functionally equivalent or protein homologous thereto are preferred.

The protein consisting of the amino acid sequence of SEQ ID NO: 6 in the sequence listing consists of 83 amino acid residues. The protein consisting of the amino acid sequence of SEQ ID NO: 6 in the sequence listing has a homology of approximately 27% in the amino acid sequence with mouse NADH-ubiquinoneoxidoreductase MLRQ subunit (CI-MLRQ). It is reported that the mouse NADH-ubiquinoneoxidoreductase MLRQ subunit exists in the complex I, one of four complexes I, II, III, and IV which form an electron transport system of a mitochondrion, and takes part in a production of an active oxygen [Circulation Res., 85, 357-363 (1999); Biochem. Mol. Biol. Int., 43, 669-675 (1997)]. The protein consisting of the amino acid sequence of SEQ ID NO: 6 in the sequence listing does not have a gap, as suggested from the amino acid sequence thereof, and it is assumed that the protein has a structure similar to that of the mouse NADH-ubiquinoneoxidoreductase MLRQ subunit. Therefore, the protein may contain an electron transport activity and take part in a production of an active oxygen upon inflammation.

Further, a signal peptide sequence at the N-terminus does not exist, and thus, it is believed that the protein consisting of the amino acid sequence of SEQ ID NO: 6 in the sequence listing exhibits biological activities in cells.

As the protein comprising the amino acid sequence of SEQ ID NO: 6 in the sequence listing, there may be mentioned, for example, a fusion protein of the protein consisting of the amino acid sequence of SEQ ID NO: 6 in the sequence listing with a fusion partner. In the fusion protein, the fusion partner may be linked with the N-terminus and/or C terminus of the protein consisting of the amino acid sequence of SEQ ID NO: 6.

As the fusion partner, for example, a protein for purification such as the whole or a part of glutathione-S-transferase (GST), a protein for detection such as the whole or a part of (β-galactosidase α peptide (LacZ α), or a protein for expression such as a signal sequence may be used.

Further, in the fusion protein, an amino acid sequence which may be restrictively digested with a proteolytic enzyme such as thrombin or factor Xa may be optionally inserted between the protein consisting of the amino acid sequence of SEQ ID NO: 6 in the sequence listing and the fusion partner.

The fragment of the protein comprising the amino acid sequence of SEQ ID NO: 6 in the sequence listing or the variation functionally equivalent or protein homologous thereto is not particularly limited, so long as it may be used as an immunogen to prepare the third antibody or fragment thereof according to the present invention, but preferably consists of 13 or more amino acid residues, more preferably 20 or more amino acid residues, most preferably 50 or more amino acid residues.

The third novel protein of the present invention may be obtained by various known methods. For example, the protein may be prepared by using a known genetic engineering technique and the third novel gene of the present invention.

The third novel gene of the present invention is not particularly limited, so long as it encodes the third novel protein of the present invention. As the gene, there may be mentioned, for example, a gene consisting of the 56th to 304th bases in the base sequence of SEQ ID NO: 5 in the sequence listing.

The gene consisting of the 56th to 1304th bases in the base sequence of SEQ ID NO: 5 in the sequence listing encodes the protein consisting of the amino acid sequence of SEQ ID NO: 6 in the sequence listing. Further, the gene consisting of the 56th to 1304th bases in the base sequence of SEQ ID NO: 5 in the sequence listing is not expressed in healthy persons, but is expressed in patients suffering from a bacterial infection.

The third probe of the present invention is not particularly limited, so long as it is capable of specifically hybridizing to an mRNA consisting of the base sequence of SEQ ID NO: 5 in the sequence listing. As the probe, there may be mentioned, for example, a single or double stranded polynucleotide consisting of a base sequence complementary to that of SEQ ID NO: 5 in the sequence listing, or a partial base sequence thereof. The lower limit of the number of bases in the third probe of the present invention is not particularly limited, but is preferably 18 or more, more particularly 26 or more, most particularly 41 or more. Further, the upper limit thereof is not particularly limited, but is preferably 652 or less. The expression "specifically hybridize with an mRNA consisting of the base sequence of SEQ ID NO: 5 in the sequence listing" as used herein means that a polynucleotide does not hybridize with mRNAs derived from a healthy person, but will hybridize with the mRNA consisting of the base sequence of SEQ ID NO: 1 in the sequence listing, under the conditions described in Example 1(4).

The plasmid of the present invention is not particularly limited, so long as it comprises the novel gene of the present invention, i.e., the first novel gene of the present invention, the second novel gene of the present invention, or the third novel gene of the present invention. For example, it may be a respective plasmid prepared by incorporating each of the above genes of the present invention into a known vector suitably selected depending on a host cell used, that is, the first plasmid of the present invention comprising the first novel gene of the present invention, the second plasmid of the present invention comprising the second novel gene of the present invention, and the third plasmid of the present invention comprising the third novel gene of the present invention.

The transformant of the present invention is not particularly limited, so long as it comprises the plasmid of the present invention, i.e., the first plasmid of the present invention, the second plasmid of the present invention, or the third plasmid of the present invention. For example, it may be a transformant prepared by transforming a desired host cell with each of the plasmids of the present invention, that is, the first transformant comprising the first plasmid of the present invention, the second transformant comprising the second plasmid of the present invention, or the third transformant comprising the third plasmid of the present invention.

The host cell may be, for example, a known microorganism usually used, for example, E. coli or Saccharomyces cerevisiae, or a known cultivated cell, such as an animal cell, such as a CHO cell or a COS cell, or an insect cell such as a BmN4 cell.

The known expression vector may be, for example, pUC, pTV, pGEX, pKK, or pTrcHis for E. coli; pEMBLY or pYES2 for a yeast; pMAMneo for a CHO cell; pcDNA3 for a COS cell; a vector (such as pBK283) containing a polyhedrin promoter of a silkworm nucleopolyhederovirus (BmNPV).

The first antibody of the present invention or the fragment thereof is reacted specifically with the first protein of the present invention or the variation functionally equivalent thereto, respectively. The second antibody of the present invention or the fragment thereof is reacted specifically with the second protein of the present invention or the variation functionally equivalent thereto, respectively. The third antibody of the present invention or the fragment thereof is reacted specifically with the third protein of the present invention or the variation functionally equivalent thereto, respectively.

The antibody of the present invention may be a monoclonal antibody or a polyclonal antibody.

The respective monoclonal antibodies of the present invention, that is, the first monoclonal antibody of the present invention specifically reactive with the first protein or the variation functionally equivalent thereto of the present invention, respectively, the second monoclonal antibody of the present invention specifically reactive with the second protein or the variation functionally equivalent thereto of the present invention, respectively, or the third monoclonal antibody of the present invention specifically reactive with the third protein or the variation functionally equivalent thereto of the present invention, respectively, may be prepared by a method which is in itself known, except that the novel protein of the present invention, the variation functionally equivalent thereto, or a fragment thereof is used as an immunogen or an antigen for a screening.

For example, a hybridoma secreting the monoclonal antibody of the present invention may be prepared by immunizing a mouse with the above immunogen, fusing a splenic cell taken from the immunized mouse and a mouse myeloma cell in accordance with a cell-fusion method disclosed in Nature, 256, 495 (1975), or an electric cell-fusion method disclosed in J. Immunol. Method, 100, 181-189 (1987), and carrying out a screening with the antigen for a screening as above.

As the medium for cultivating the hybridomas, any medium suitable for a cultivation of a hybridoma may be used. Preferably, the Dulbecco's modified Eeagle's minimum essential medium containing fetal calf serum, L-glutamine, L-pyruvic acid, and antibiotics (penicillin G and streptomycin) may be used.

The cultivation of the hybridoma may be carried out in 5 % $CO_2$ and at 37° C for about 3 days in a medium, or for about 14 days in the abdominal cavities of mice.

It is possible to isolate or purify the monoclonal antibody from the resulting culture liquid or mouse ascites, using a method generally applied for the isolation and purification of proteins.

As examples thereof, there may be mentioned ammonium sulfate salting out, ion exchange column chromatography using ion exchange cellulose, molecular sieve column chromatography using molecular sieve gel, affinity column chromatography using protein A binding polysaccharides, dialysis, lyophilization, or the like.

The respective polyclonal antibodies of the present invention, that is, the first polyclonal antibody of the present invention specifically reactive with the first protein or the variation functionally equivalent thereto of the present invention, respectively, the second polyclonal antibody of the present invention specifically reactive with the second protein or the variation functionally equivalent thereto of the present invention, respectively, or the third polyclonal antibody of the present invention specifically reactive with the third protein or the variation functionally equivalent thereto of the present invention, respectively, may be also prepared by a method which is in itself known, except that the novel protein of the present invention, the variation functionally equivalent thereto, or a fragment thereof is used as an immunogen or an antigen for a screening by, for example, the following method.

That is, a physiological salt solution containing an antigen is mixed with an equal volume of complete Freund's adjuvant or incomplete adjuvant, or an equivalent thereof, such as Hunter's TiterMax™ (Funakoshi; Cat. No. YT001-00, Tokyo, Japan), until emulsified. The resulting emulsion is administered subcutaneously, intraperitoneally, or intramuscularly to a mammal, for example, a rabbit or goat (a first immunization). Then, the same procedure is repeated at intervals of two to four weeks for several immunizations. One or two weeks after a final immunization, blood is taken from a carotid artery or a heart of the mammal, and salted-out with ammonium sulfate to prepare a serum.

Each of the antibody-fragments of the present invention is not particularly limited, so long as it is a partial fragment of the antibody (including the monoclonal antibody and the polyclonal antibody) of the present invention, and has a specific reactivity that is the same as that of the original antibody. The fragment of the present invention may be, for example, Fab, Fab', $F(ab')_2$, or Fv. The antibody fragment of the present invention may be prepared, for example, by digesting the polyclonal antibody or monoclonal antibody of the present invention with a known protease by a conventional method, and then isolating and purifying by a conventional method.

The inventors of the present invention found that the protein of the present invention, particularly, the protein consisting of the amino acid sequence of SEQ ID NO: 2 in the sequence listing, the protein consisting of the amino acid sequence of SEQ ID NO: 4 in the sequence listing, or the protein consisting of the amino acid sequence of SEQ ID NO: 6 in the sequence listing, and the mRNA thereof, is not expressed in a healthy person but in a patient suffering from a bacterial infection, such as septicemia, pneumonia, urinary tract infection, myelitis, or tympanitis. Therefore, the proteins of the present invention or mRNAs thereof may be used as a diagnostic marker of a patient suffering from a bacterial infection. More particularly, when an *in vitro* detection method of the present invention finds an existence of the protein of the present invention and/or mRNA thereof in a sample taken from a subject, the subject can be judged to be a patient suffering from a bacterial infection. On the contrary, when the protein and/or the mRNA is not found, the subject can be judged to be a person not suffering from a bacterial infection.

The sample which may be used in the present invention is not particularly limited, so long as it has a possibility of including the protein of the present invention and/or the mRNA thereof. The sample may be a biological sample taken from an animal, such as a mammal, particularly a human (particularly a patient), for example, a tissue (e.g., cells) or an extract therefrom, blood such as serum or plasma, urine, or a humor such as cerebrospinal fluid. A sample used in a conventional clinical examination may be used in the present invention without limitation.

The method of the present invention will be explained hereinafter with respect to the method for detecting a bacterial infection by analyzing the mRNA of the protein of the present invention, and then, with respect to the method for detecting a bacterial infection by analyzing the protein of the present invention.

In the methods of the present invention, the method for detecting a bacterial infection by analyzing the mRNA of the protein of the present invention is not particularly limited but, for example, may be a method comprising steps of bring a sample into contact with a polynucleotide comprising a base sequence complementary to the base sequence of the mRNA of the protein of the present invention; and analyzing a coupled product of the polynucleotide and the mRNA of the protein of the present invention (hereinafter referred to as a "first detecting method of the present invention"), or a method comprising the steps of reverse-transcribing an mRNA in a sample to a cDNA, amplifying genes in accordance with a gene-amplifying reaction, particularly, a polymerase chain reaction (PCR), using a reaction product obtained in the reverse-transcribing step, and primers which may amplify genes with the gene encoding the present protein as a template, and analyzing the amplified genes in the above gene-amplifying step (hereinafter referred to as a "second detecting method of the present invention").

In the first method for detection of the present invention, the sample is reacted with a polynucleotide (for example, the probe of the present invention) comprising a base sequence complementary to that of the mRNA of the protein of the present invention, and the resulting complex of the polynucleotide and "the mRNA of the protein of the present invention" is detected, or the amount of the complex is measured to thereby analyze the mRNA of the protein of the present invention.

The polynucleotide comprises a sequence complementary or substantially complementary to that of a part of the mRNA transcribed from a selected gene (DNA), and thus forms a double strand with the mRNA transcribed from the target gene. It is believed that any polynucleotide sufficiently complementary to form a stable complex with a target mRNA can be used. The polynucleotide able to be used in the present invention may be complementary to substantially any region in a target mRNA. The polynucleotide can be used as a DNA probe for detecting an increase or a decrease of an expression of the mRNA specific to the gene of the protein according to the present invention. That is, the polynucleotide is specifically attached to the mRNA of the protein according to the present invention as a target, and forms a molecular hybrid, whereby a degree of expression of the mRNA of the protein according to the present invention in cells can be detected.

The polynucleotide able to be used in the first method for detection of the present invention may be prepared by appropriately selecting a base sequence complementary to a specific base sequence of the mRNA of the protein according to the present invention, and using a known DNA synthesizer, a PCR apparatus, a gene cloning or the like. Various length polynucleotides may be used, but the polynucleotide preferably has 10 or more bases, more preferably 17 or more bases.

The polynucleotide may be a non-modified polynucleotide or a polynucleotide analogue. An appropriate analogue may be, for example, an ethyl or methyl phosphate analogue, or a phosphorothioated polydeoxynucleotide [Nucleic Acids Res., 14, 9081-9093, (1986); J. Am. Chem. Soc., 106, 6077-6079, (1984)], with recent improvement in the production of polynucleotide analogue, for example, a 2'-O-methylribonucleotide [Nucleic Acids Res., 15, 6131-6148, (1987)], or a conjugated RNA-DNA analogue, i.e., chimera polynucleotide [FEBS Lett., 215, 327-330, (1987)], may be used.

The selected polynucleotide may be of any kind, for example, may have an electrical charge or no electrical charge. The polynucleotide may be labeled with a known labeling agent, such as a radioactive isotope, or a fluorescent substance by a conventional method, so as to carry out the above experiment in vitro or in vivo. The radioactive isotope may be, for example, $^{125}I$, $^{131}I$, $^{3}H$, $^{14}C$, $^{32}P$, or $^{35}S$. Of these radioactive isotopes, it is preferable to label the polynucleotide with $^{32}P$ by a random primer method [Anal. Biochem., 132, 6-13, (1983)]. Further, a fluorescent coloring agent forming a derivative may be used as a labeling agent, as this enables an easy handling with a low risk factor. As the fluorescent coloring agent, any coloring agents capable of binding the polynucleotide may be used. For example, fluorescein, rhodamin, Texas red, 4-fluoro-7-nitrobenzofurazane (NBD), coumarin, fluorescamine, succinyl fluorescein, or dansyl may be preferably used.

An amount of an mRNA of the protein according to the present invention may be measured by a northern blotting method, using cDNA of the protein according to the present invention as follows: an mRNA is extracted and isolated from any somatic cell or tissue, then the isolated mRNA is electrophoresed on an agarose gel and transferred onto a nitro cellulose or nylon membrane, and then reacted with a cDNA probe of the protein according to the present invention to measure an amount of the mRNA of the protein according to the present invention. The cDNA probe of the protein according to the present invention as used is a DNA complementary to the mRNA of the protein according to the present invention, and has preferably 17 or more bases.

In the reverse-transcribing step and the gene-amplifying step (particularly, the PCR step) of the second detecting method according to the present invention, the reactions per se may be carried out in accordance with the conventional reverse-transcribing method and the conventional gene-amplifying method, for example, a reverse-transcription PCR (RT-PCR). More particularly, a reverse-transcriptase and oligo(dT) primers are used to carry out the reverse-transcription. Then, a thermostable DNA polymerase, such as a Taq polymerase, is used to carry out an initial denaturing reaction, for example at 97 °C for 2 to 3 minutes. Subsequently, an amplifying cycle consisting of (1) a step for denaturing DNAs at 90 to 94 °C for 30 seconds, (2) a step for annealing single-strand DNAs and primers at 50 to 55 °C for 30 seconds, and (3) a step for synthesizing DNAs by the thermostable DNA polymerase at 70 to 75 °C for 1 to 2 minutes is repeated, for example 15 to 45 times, to perform the PCR.

The analyzing step of the second detecting method according to the present invention can be carried out in accordance with, for example, a conventional analyzing method, for example, a method comprising steps of carrying out agarose-gel electrophoresis and then staining the gel with a suitable DNA-binding colorant such as ethidium bromide, or a southern blotting, or the like

The method for detecting a bacterial infection by analyzing the protein of the present invention, one of the methods according to the present invention, is not particularly limited but, for example, may comprise steps of bringing a sample into contact with an immunoreactive substance which can immunologically react with the protein of the present invention, and analyzing a coupled product of the immunoreactive substance and the protein of the present invention (hereinafter referred to as a "third detecting method of the present invention").

In the third detecting method of the present invention, the sample is brought into contact with the immunoreactive substance which may immunologically react with the protein of the present invention. When a sample from a human is used, the sample is preferably brought into contact with an immunoreactive substance which can immunologically react with the protein consisting of the amino acid sequence of SEQ ID NO: 2, the protein consisting of the amino acid sequence of SEQ ID NO: 4, or the protein consisting of the amino acid sequence of SEQ ID NO: 6.

When the sample is brought into contact with the substance immunologically reactive to the protein of the present invention, if the sample does not contain the protein of the present invention, a reaction with the immunologically reactive substance does not occur. If the sample contains the protein of the present invention, the immunologically reactive substance binds the protein of the present invention, and a complex of the immunologically reactive substance and the protein of the present invention is formed in an amount correlated with that of the protein of the present invention present in the sample. The complex may be easily detected by a known method, and therefore, an existence of the protein of the present invention in the sample can be detected by detecting the existence of the complex, or an amount of the protein of the present invention in the sample can be measured by measuring the amount of the complex. The protein of the present invention in a tissue or a cell may be measured by using a tissue section sample or a cell sample in a fluorescent antibody technique or an enzyme antibody technique.

The immunologically reactive substance capable of immunologically reacting the protein of the present invention includes an antiserum against the protein of the present invention, a polyclonal antibody against the protein of the present invention, or a monoclonal antibody against the protein of the present invention, or a fragment of these antibodies. The immunologically reactive substance may be used singly or in a combination thereof. The fragment includes, for example, Fab, Fab', $F(ab')_2$, or Fv.

In the third method for detection according to the present invention, the sample is brought into contact with the immunologically reactive substance capable of immunologically reacting the protein of the present invention, and a complex of the protein in the present invention and the immunologically reactive substance is formed. Then, the protein in the present invention bound to the antibody is detected and the amount thereof is measured by an immunochemical method, to thereby find a level of the protein of the present invention in the sample.

Principally, the immunochemical method may be, for example, any conventional immunoassay, for example, EIA, ELISA, RIA or the like. The immunochemical methods are generally classified as follows:

(1) Competitive assay:

A sample containing an unknown amount of antigens and a given amount of labeled antigens is competitively reacted with a given amount of antibodies, and then an activity of the labeled antigens bound to the antibodies or an activity of the labeled antigens not bound to the antibodies is measured.

(2) Sandwich assay:

An excess amount of antibodies immobilized on carriers is added and reacted to a sample containing an unknown amount of antigens (a first reaction). Then, a given excess amount of labeled antibodies is added and reacted therewith (a second reaction). An activity of the labeled antibodies on the carriers is measured.

Alternatively, an activity of the labeled antibodies which are not on the carriers is measured. The first reaction and the second reaction may be carried out at the same time, or sequentially.

When a labeling agent is a radioactive isotope, a well counter or a scintillation counter may be used for measurement. When the labeling agent is an enzyme, an enzymatic activity can be measured by colorimetry or fluorimetry, after adding a substrate and allowing to stand. When the labeling agent is a fluorescent substance or an luminescent substance, a known method therefor may be used, respectively.

Recently, in addition to the above methods, a western blotting method has been used wherein electrophoresed proteins are transferred onto a filter such as a nitrocellulose membrane, and a target protein is detected with an antibody. The western blotting method may also be used in the detection of the protein according to the present invention.

The antibody used in the above methods can be labeled with an appropriate marker. Examples are a radioactive isotope, an enzyme, a fluorescent substance, or a luminescent substance, by a known method of labeling antibodies.

The radioactive isotope may be, for example, $^{125}I$, $^{131}I$, $^{3}H$, $^{14}C$, or $^{35}S$.

Preferably, the enzyme used is stable and has a large specific activity. Examples of the enzyme are a glycosidase (such as, β-galactosidase, β-glucosidase, β-glucuronidase, β-fructosidase, α-galactosidase, α-glucosidase, or α-mannosidase), an amylase (such as, α-amylase, β-amylase, isoamylase, glucoamylase, or taka-amylase), a cellulase, or a carbohydrase such as lysozyme; a urease, or an amidase such as asparaginase; a choline esterase, such as acetylcholinesterase, a phosphatase, such as alkaline phosphatase, a sulfatase, an esterase such as lipase; a nuclease such as deoxyribonuclease or ribonuclease; an iron porphyrin enzyme, such as a catalase, peroxidase or cytochrome oxidase; a copper enzyme, such as a tyrosinase or ascorbate oxidase; dehydrogenase, such as an alcohol dehydrogenase, malate dehydrogenase, lactate dehydrogenase, or isocitrate dehydrogenase.

The fluorescent substance may be, for example, fluorescamine, or a fluorescence isothiocyanate, and the luminescent substance may be, for example, luminol, a luminol derivative, luciferin or lucigenin. A signal from the above label may be detected by known methods.

The labeling agent can be bound to antibodies by any conventional method, such as a chloramin T method [Nature, 194, 495-496, (1962)], a periodic acid method [Journal of Histochemistry and Cytochemistry, 22, 1084-1091, (1974)], or a maleimide method [Journal of Biochemistry, 79, 233-236, (1976)].

An EIA method, as one of the above measurement methods will be mentioned hereinafter. A sample is added to the first antibodies immobilized on a carrier (such as an assay plate), and the first antibodies are bound to the proteins of the present invention to form complexes. To the complexes, the second antibodies labeled with enzyme (such as peroxidase) are added to react with the complexes to form "first antibody/protein of the present invention/second antibody" complexes. To the resulting "first antibody/protein of the present invention/second antibody" complexes, a substrate for the enzyme label (such as peroxidase) is added, and an absorbance or fluorescent strength of products of the enzymatic reaction is measured, whereby enzymatic activities of the enzyme labels attached to the "first antibody/protein in the present invention/second antibody" complexes are measured. A series of the above procedures is carried out in advance for a standard solution containing a known amount of the protein of the present invention, and a standard curve based on the relationship between the protein of the present invention and the absorbance or fluorescent strength is prepared. A comparison is made between the standard curve and absorbance or fluorescent strength for a sample containing an unknown amount of the proteins according to the present invention, and thus, the amount of the proteins according to the present invention in the sample can be measured.

Another EIA method will be mentioned hereinafter. A sample is brought into contact with a carrier (such as an assay plate) to immobilize the proteins of the present invention in the sample on the carrier. Then, the first antibodies are added thereto to form complexes of the protein according to the present invention and the first antibody. To the complexes are added anti-first antibody antibodies (second antibodies) labeled with an enzyme (such as peroxidase), to react with the complexes to form "protein of the present invention/first antibody/second antibody" complexes. To the resulting "protein of the present invention/first antibody/second antibody" complexes is added a substrate for the enzyme label (such as peroxidase), and the absorbance or fluorescent strength of products of the enzymatic reaction is measured, whereby enzymatic activities of the enzyme labels attached to the "protein of the present invention/first antibody/second antibody" complexes are measured. A series of the above procedures is carried out in advance for a standard solution containing a known amount of the protein according to the present invention, and a standard curve based on the relationship between the protein of the present invention and the absorbance or fluorescent strength is prepared. A comparison is made between the standard curve and the absorbance or fluorescent strength for a sample containing an unknown amount of the proteins according to the present invention, and the amount of the proteins according to the present invention in the sample is measured.

Further, an RIA method will be mentioned hereinafter. A sample is added to the first antibodies immobilized on a carrier (such as a test tube), and the first antibodies are bound to the proteins of the present invention to form complexes. To the complexes are added the second antibodies labeled with radioactive isotope (such as $^{125}I$), to react with the complexes to form "first antibody/protein of the present invention/second antibody" complexes. A radioactivity (such as γ-radioactivity) of the resulting "first antibody/protein of the present invention/second antibody" complexes is measured. A series of the above procedures is carried out in advance for a standard solution containing a known amount of the protein according to the present invention, and a standard curve based on the relationship between the protein of the present invention and the radioactivity is prepared. A comparison is made between the standard curve and the radioactivity for a sample containing an unknown amount of the proteins according to the present invention, and the amount of the proteins according to the present invention in the sample is measured.

Another RIA method will be mentioned hereinafter. A sample is brought into contact with a carrier (such as a test tube) to immobilize the proteins of the present invention in the sample on the carrier. Then, the first antibodies are added thereto to form complexes of the protein according to the present invention and the first antibody. To the complexes are added anti-first antibody antibodies (second antibodies) labeled with a radioactive isotope (such as $^{125}I$), to react with the complexes to form "protein of the present invention/first antibody/second antibody" complexes. A radioactivity (such as γ-radioactivity) of the resulting "protein of the present invention/first antibody/second antibody" complexes is measured. A series of the above procedures is carried out in advance for a standard solution containing a known amount of the protein according to the present invention, and a standard curve based on the relationship between the protein in the present invention and the radioactivity is prepared. A comparison is made between the standard curve and the radioactivity for a sample containing an unknown amount of the proteins according to the present invention, and the amount of the proteins according to the present invention in the sample is measured.

EXAMPLES

The present invention now will be further illustrated by, but is by no means limited to, the following Examples.

Example 1: Isolation and identification of activated human macrophage-specific novel genes

(1) Preparation of mRNA derived from macrophages stimulated by lipopolysaccharide (LPS)

From 1 liter of blood derived from a healthy person as a starting material, peripheral blood monocytes were prepared using a commercially available reagent for preparing peripheral blood monocytes (Lymphoprep; Nycomed, Oslo, Norway). The obtained peripheral blood monocytes were suspended in an RPM1640 medium containing 10 μg/mL LPS (Difco Laboratories, Detroit, MI, USA) and 10% fetal calf serum (FCS) so that the concentration of cells became $10^6$ cells/mL. To each plastic dish, 20 mL of the cell suspension was poured and cultured under the condition of 37°C and 5% $CO_2$.

After culturing for 3 hours, the supernatant was discarded, and adherent cells (i.e., macrophages stimulated by LPS) were washed three times with 20 mL of a phosphate-buffered saline (PBS). After 3 mL of a solution for cell lysis [4 mol/L guanidine isothiocyanate, and 30 mmol/L sodium acetate (pH 4.8)] was added, suction and ejection were repeated three times using a syringe with a needle. The lysate was put on 1.2 mL of a 5.7 mol/L cesium chloride buffer (pH 4.8) in a ultracentrifuge 5PA tube (Hitachi Koki; Katsuta, Japan). After centrifuging for 18 hours (20°C, 38000 rpm), the supernatant in the centrifuge tube was discarded. The pellet in the centrifuge tube was dissolved in 200 μL of sterile water to collect RNA. From 1L of blood, approximately 1 mg of total RNA (i.e., total RNA derived from macrophages stimulated by LPS) was obtained.

Then, using a commercially available kit for preparing mRNA [Poly(A) Quik mRNA Isolation Kit; Stratagene, La Jolla, CA, USA], 15 μg of mRNA (i.e., mRNA derived from macrophages stimulated by LPS) was prepared from 500 μg of the total RNA.

(2) Preparation of a phage cDNA library

A phage cDNA library was prepared using 5 μg of the obtained mRNA (15 μg) derived from macrophages stimulated by LPS. Commercially available kits (ZAP Express cDNA Synthesis Kit and ZAP Express cDNA Gigapack III Gold Cloning Kit; Stratagene) were used to prepare the phage cDNA library.

(3) Analysis of partial base sequences of cDNAs

To analyze base sequences of cDNAs derived macrophages stimulated by LPS, approximately 1000 phage plaques were picked up at random and cDNAs were recovered as a phagemid by a conventional method. With respect to the recovered approximately 1000 cDNAs derived macrophages stimulated by LPS, 400 to 500 bases from the 5' terminus and 3' terminus of the cDNAs were analyzed, respectively, using a commercially available kit for determining base sequences (Dye Terminator Cycle Sequencing kit; Perkin Elmer Japan, Urayasu). The DAN homology search of the obtained sequences was carried out using BLAST (basic local alignment tool) in NCBI (National Center for Biotechnology Information; http://inhouse.ncbi.nlm.nih.gov) and found 63 unknown novel genes.

(4) Analysis by northern blotting

Total RNA derived macrophages stimulated by LPS were prepared in accordance with the procedure for preparing total RNA derived macrophages stimulated by LPS described in Example (1). Further, total RNAs derived macrophages not stimulated by LPS were prepared in accordance with the procedure for preparing total RNA derived macrophages stimulated by LPS described in Example (1), except for using an RPM1640 medium containing 10% FCS instead of the RPM1640 medium containing 10 µg/mL LPS and 10% FCS. The total RNA derived macrophages stimulated by LPS (10 µg/mL LPS) and the total RNA derived macrophages not stimulated by LPS were electrophoresed on a formaldehyde/agarose gel and transferred onto a nylon membrane filter by a conventional method.

The filter onto which the RNAs were transferred was heat-treated at 80°C under reduced pressure for 2 hours, and immersed in a commercially available solution for prehybridization (Hybrisol I; Oncor, Gaithersburg, MD, USA) to perform prehybridization at 42°C for 3 hours. Then, the novel genes obtained in Example 1(3) labeled with isotope $^{32}P$ using a random primed labeling kit (Boehringer Mannheim; Germany) were respectively added, and hybridization was carried out overnight at 42°C. On the next day, the filter was twice washed with 2xSSC (standard sodium citrate) containing 0.1% sodium dodecyl sulfate (SDS) at room temperature for 20 minutes, and further, twice washed with 0.2xSSC containing 0.1% SDS at 65°C for 20 minutes. The washed filter was wrapped in a wrap, and autoradiography was performed overnight at -80°C.

As a result, it was found that, among 63 novel genes obtained in Example 1(3), three genes were those whose expression was induced by the LPS-stimulation. The results of northern blotting with respect to the three novel genes (NLG-1-1, NLG-1-2, and NLG-2) are shown in Fig. 1. In Fig. 1, the symbol "+" means "stimulated by LPS", the symbol "-" means "not stimulated by LPS", and "Origin" means "starting point of electrophoresis". The lengths of the mRNAs of the three novel genes (NLG-1-1, NLG-1-2, and NLG-2) were approximately 2.3 kb, approximately 2.3 kb, and approximately 0.7 kb, respectively.

Further, the tissue-specific expression was examined. As shown in Fig. 2, the genes NLG-1-1 and NLG-1-2 were weakly expressed in all tissues examined [i.e., spleen (lane 1), thymus (lane 2), prostate (lane 3), testis (lane 4), ovary (lane 5), small intestine (lane 6), large intestine (lane 7) and peripheral blood lymphocyte (lane 8)]. On the contrary, the gene NLG-2 was strongly expressed in testis (lane 4) and large intestine (lane 7), but not expressed in the other tissues.

(5) Determination of full-length base sequences

The full-length base sequences of the three novel genes (LG-1-1, NLG-1-2, and NLG-2) were determined by a conventional method.

The genes NLG-1-1 and NLG-1-2 consist of 2180 bp and 1970 bp, respectively. The concrete base sequences thereof are those of SEQ ID NO: 1 and SEQ ID NO: 3 in the sequence listing, respectively. As the result of a homology search of the genes NLG-1-1 and NLG-1-2, the 193rd to 2139th base sequence of the gene NLG-1-1 was found to be completely identical to the 9th to 1955th base sequence of the gene NLG-1-2. It is presumed that two mRNAs are transcribed from a chromosomal gene in accordance with an alternative splicing. The gene NLG-1-1 encodes a protein consisting of 481 amino acid residues having the amino acid sequence of SEQ ID NO: 2 in the sequence listing. The gene NLG-1-2 encodes a protein consisting of 390 amino acid residues having the amino acid sequence of SEQ ID NO: 4 in the sequence listing.

Further, the gene NLG-2 consists of 652 bp, and the concrete base sequence thereof is that of SEQ ID NO: 5 in the sequence listing. The gene NLG-2 encodes a protein consisting of 83 amino acid residues having the amino acid sequence of SEQ ID NO: 6 in the sequence listing.

Example 2: Expression of the genes NLG-1-1 and NLG-2 in an animal cell

In this example, the genes NLG-1-1 and NLG-2 were expressed using COS-1 (Dainippon pharmaceutical, Suita, Osaka, Japan) as an animal cell and a pQBI25-fN3rsGFP vector (Quantum biotechnologies, Montreal, Quebec, Canada) by the following procedure. This was because when the above vector is used, a desired gene can be expressed in the form of a fused protein with a green fluorescent protein (GFP), and therefore, locations of the desired gene products can be observed by tracing the green fluorescence.

Each cDNA of genes NLG-1-1 and NLG-2 was prepared by a reverse transcription PCR (RT-PCR) method in accordance with the following procedure. An mRNA was prepared from human peripheral blood monocytes (PBMC) stimulated by LPS for 3 hours. A cDNA synthesized from the mRNA using a commercially available cDNA synthesis kit (SMART PCR cDNA synthesis kit; Clontech, Palo alto, CA, USA) was used as a template.

As primers, an NLG-2 forward primer consisting of the sequence:

5'-CACGGATCCATTCTTCGCTGAAGTCATCATGAGC-3'(SEQ ID NO: 7),

an NLG-2 reverse primer consisting of the sequence :

5'-GTGGAATTCTTTGGTCACCCTTTGGACATTTTGC-3'(SEQ ID NO: 8),

an NLG-1-1 forward primer consisting of the sequence :

5'-CACGGATCCTTCTTTACAACGAAATGATGCTCAAG-3'(SEQ ID NO: 9),

and an NLG-1-1 reverse primer consisting of the sequence :

5'-GTGGAATTCGGAGAGATTTGTGATAGAATTATTACATGC-3'(SEQ ID NO: 10)

were used.

Using a commercially available reagent for PCR (Advantage cDNA polymerase Mix; Clontech), PCR was carried out by repeating a cycle consisting of a denaturation step (94°C, 30 seconds) and an annealing and elongation step (68°C, 2 minutes), 30 times.

The obtained PCR product was digested with restriction enzymes BamHI (Takarashuzo, Chuo-ku, Tokyo, Japan) and EcoRI (Takarashuzo), and cloned in a pQBI25-fN3rsGFP vector using a commercially available kit (DNA ligation kit Ver. 2; Takarashuzo) to use for the following experiments.

On the day before a gene transfer, COS-1 cells were plated on a 6-well plate so that the concentration of cells became $1x10^6$ cells/well. In each well of the 6-well plate, an autoclaved cover glass was placed, and cells were cultured on the cover glass. On the next day, the previously obtained vector was transferred to cos-1 cells using a commercially available reagent for transfection (LipofectAMINE reagent; Gibco BRL, Rockville, MD, USA). After 3 days from the transfer, cells on the cover glass were fixed in PBS containing 4% (v/v) formalin for 30 minutes, treated in PBS containing 0.2% (v/v) Triton X-100 for 30 minutes, and treated in a blocking reagent (Block Ace; Dainippon pharmaceutical) containing 20% (v/v) normal goat serum (Vector Laboratories, Burlingame, CA, USA) for 30 minutes.

For immunostaining a mitochondrion, an anti-cytochrome c antibody (Santa Cruz Biotechnology, Santa Cruz, CA, USA) and a Texas red-labeled anti-rabbit IgG antibody (Vector Laboratories) were used. For immunostaining an endoplasmic reticulum, an anti-calreticulin antibody (Upstate Biotechnology, Lake Placid, NY, USA) and a Texas red-labeled anti-rabbit IgG antibody (Vector Laboratories) were used. For immunostaining a Golgi apparatus, an anti-Golgi 58K protein antibody (Sigma, St. Louis, MO, USA) and a Texas red-labeled anti-mouse IgG antibody (Kirkegaard & Perry Laboratories, Gaithersburg, MD, USA) were used.

For staining a nucleus, propidium iodide (Wako Pure Chemical Industries, Osaka, Osaka, Japan) was used. For staining cytoplasm, hydroethidine (Polysciences, Warrington, PA, USA) was used.

The cover glass was mounted on a slide glass, and observed with a confocal laser scanning microscope FV500 (Olympus Optical Company Limited, Chiyoda-ku, Tokyo, Japan). The state of COS-1 cells in which the gene NLG-1-1 was expressed is shown in Fig. 3. The state of COS-1 cells in which the gene NLG-2 was expressed is shown in Fig. 4. In Figs. 3 and 4, "A" is a green fluorograph showing the expression of the fusion protein of GFP with the protein encoded by the gene NLG-1-1 or NLG-2; "B" is a red fluorograph wherein mitochondria were stained; "C" is a combination of the above fluorographs A and B; and "D" is a figure of differential calculus interference.

As shown in Fig. 3A, the expression of the gene NLG-1-1 was observed around the nucleus. Further, the green fluorograph in Fig. 3A and the red fluorograph (the fluorograph wherein mitochondria were stained) in Fig. 3B accorded well. In Fig. 3C, the areas wherein fluorographs A and B accorded were shown in yellow. On the contrary, the fluorograph wherein endoplasmic reticula were stained, the fluorograph wherein Golgi apparatus were stained, the image wherein nuclei were stained, or the image wherein cytoplasm was stained did not accord with the green fluorograph in Fig. 3A. Accordingly, it was found that the protein encoded by the gene NLG-1-1 was localized in mitochondria.

Further, as apparent from Fig. 4, it was found that the protein encoded by the gene NLG-2 was localized in mitochondria, as the protein encoded by the gene NLG-1-1.

Example 3: Determination of the chromosomal locus of the gene NLG-1-1

The chromosomal locus of the gene NLG-1-1 was determined by a FISH (fluorescence in situ hybridization) analysis [Chromosoma., 102, 325-332 (1993)]. The gene was located on 13q22. The results were shown in Fig. 5. In Fig. 5, "A" is the result of DAPI (4',6-diamidino-2-phenylindole) staining, and "B" is the result of the FISH signal. The arrow in Fig. 5A denotes the chromosomal locus of the gene NLG-1-1, and the number "13" denotes that it is the thirteenth chromosome.

Example 4: Determination of expression of the genes NLG-1-1, NLG-1-2, and NLG-2 in patients suffering from septicemia

From 20 mL of each blood collected from four healthy persons and four patients suffering from septicemia as a starting material, peripheral blood monocytes were prepared, and then approximately 20 μg of total RNA was respectively prepared from the peripheral blood monocytes in accordance with the procedure described in Example 1(1).

Using 10 μg of each RNA, northern blotting was carried out in accordance with the procedure described in Example 1(4).

The results are shown in Fig. 6. In Fig. 6, (A) shows the result using the gene NLG-1-1 as a probe. Similarly, (B) and (C) show the results using the genes NLG-1-2 and NLG-2 as a probe, respectively. Further, in Fig. 6, lanes 1 to 4 denote the results of RNAs derived from the healthy persons, and lanes 5 to 8 denote the results of RNAs derived from the patients suffering from septicemia.

As shown in Fig. 6, three novel genes (NLG-1-1, NLG-1-2, and NLG-2) of the present invention were not expressed in all four healthy persons, but were strongly expressed in all four patients suffering from septicemia. These results show that these genes of the present invention may be used for the diagnosis of a bacterial infection or a judgment of prognosis by analyzing the expression of the genes.

INDUSTRIAL APPLICABILITY

It is known that life phenomena caused by an LPS-stimulation are similar to those in inflammation. In fact, the genes having an expression that is induced by an LPS-stimulation specifically at a macrophage playing a central role in inflammation include almost all genes encoding proteins leading to inflammatory diseases, for example, an inflammatory cytokine (TNF, IL-1, IL-6, IL-18), a chemokine (IL-8, MCP), a secretory protein, such as a collagenase abnormally produced at a diseased part of rheumatoid arthritis which is an inflammatory disease, an NO synthase which is an intracellular protein and produces NO (nitrogen monoxide) causing an inflammation, cylooxygenese (COXII) producing a prostaglandin, an NF-kB, i.e., a gene-transcription factor participating in an expression of a gene encoding an inflammatory protein, or the like.

Clinical development for inhibitors of the above proteins as an antiinflammatory drug has been intensively carried out, and many inhibitors are undergoing a clinical study. For example, an anti-TNF antibody or an inhibitor of NO synthase is already being used as a medicament for treating an inflammatory disease. Further, a clinical development of a collagenase inhibitor as an inhibitor for a cancer metastasis, and of a COXII inhibitor as an anticancer drug is already being carried out. These suggest that there is a strong possibility that the novel genes having an expression that is induced by an LPS-stimulation and proteins according to the present invention also participate in the outbreak and/or deterioration of an inflammatory disease, an allergy disease, or a cancer.

The possibility exists that the first and second novel proteins of the present invention take part in an intracellular signal transduction system of an LPS. Further, the possibility exists that the third novel protein of the present invention takes part in an intracellular electron transportation and/or a radical production. Therefore, the proteins as above are different from a target of a conventional development of an antiinflammatory drug, whereas inhibitors of the proteins as above will be a new type of antiinflammatory drug. Further, an examination of an expression of the genes encoding the proteins as above in a human clinical sample by means of a reverse transcription PCR (RT-PCR), a northern blotting, a dot blotting, or a DNA microarray would make it possible to carry out a diagnosis of an inflammation, an allergy, or a cancer. Furthermore, an antibody against the protein as above could be used to carry out a diagnosis of an inflammation, an allergy, or a cancer. Still further, antisense DNAs of the genes encoding the proteins as above could be used in a treatment of an inflammation, an allergy, or a cancer (including a gene therapy).

The probe or the antibody according to the present invention may be used to carry out a diagnosis of a bacterial infection, such as septicemia, pneumonia, urinary tract infection, myelitis, or tympanitis, or a judgment of prognosis. The proteins of the present invention are useful for preparing the antibodies of the present invention, and the novel genes, plasmids, and transformants of the present invention are useful for preparing the proteins of the present invention.

As above, the present invention is explained with reference to particular embodiments, but modifications and improvements obvious to those skilled in the art are included in the scope of the present invention.

**Claims**

1. A protein comprising an amino acid sequence of SEQ ID NO: 2 in the sequence listing, or a variation functionally equivalent thereto, or a fragment of said protein or said variation.

2. A gene encoding said protein or said variation functionally equivalent thereto according to claim 1.

3. The gene according to claim 2, consisting of the 37th to 1479th bases in a base sequence of SEQ ID NO: 1 in the sequence listing.

4. A plasmid comprising said gene according to claim 2 or 3.

5. A transformant comprising said plasmid according to claim 4.

6. An antibody or a fragment thereof, **characterized by** being reactive specifically to said protein or said variation functionally equivalent thereto according to claim 1.

7. A method for detecting a bacterial infection, **characterized by** analyzing a protein comprising an amino acid sequence of SEQ ID NO: 2 in the sequence listing or a variation functionally equivalent thereto, or an mRNA thereof in a sample to be detected.

8. The method according to claim 7, wherein the bacterial infection is septicemia.

9. A polynucleotide capable of specifically hybridizing to an mRNA consisting of an base sequence of SEQ ID NO: 1 in the sequence listing.

10. A protein comprising an amino acid sequence of SEQ ID NO: 4 in the sequence listing, or a variation functionally equivalent thereto, or a fragment of said protein or said variation.

11. A gene encoding said protein or said variation functionally equivalent thereto according to claim 10.

12. The gene according to claim 11, consisting of the 126th to 1295th bases in a base sequence of SEQ ID NO: 3 in the sequence listing.

13. A plasmid comprising said gene according to claim 11 or 12.

14. A transformant comprising said plasmid according to claim 13.

15. An antibody or a fragment thereof, **characterized by** being reactive specifically to said protein or said variation functionally equivalent thereto according to claim 10.

16. A method for detecting a bacterial infection,
**characterized by** analyzing a protein comprising an amino acid sequence of SEQ ID NO: 4 in the sequence listing or a variation functionally equivalent thereto, or an mRNA thereof in a sample to be detected.

**17.** The method according to claim 16, wherein the bacterial infection is septicemia.

**18.** A polynucleotide capable of specifically hybridizing to an mRNA consisting of an base sequence of SEQ ID NO: 3 in the sequence listing.

**19.** A protein comprising an amino acid sequence of SEQ ID NO: 6 in the sequence listing, or a variation functionally equivalent thereto, or a fragment of said protein or said variation.

**20.** A gene encoding said protein or said variation functionally equivalent thereto according to claim 19.

**21.** The gene according to claim 20, consisting of the 56th to 304th bases in a base sequence of SEQ ID NO: 5 in the sequence listing.

**22.** A plasmid comprising said gene according to claim 20 or 21.

**23.** A transformant comprising said plasmid according to claim 22.

**24.** An antibody or a fragment thereof, 1 **characterized by** being reactive specifically to said protein or said variation functionally equivalent thereto according to claim 19.

**25.** A method for detecting a bacterial infection,
**characterized by** analyzing a protein comprising an amino acid sequence of SEQ ID NO: 6 in the sequence listing or a variation functionally equivalent thereto, or an mRNA thereof in a sample to be detected.

**26.** The method according to claim 25, wherein the bacterial infection is septicemia.

**27.** A polynucleotide capable of specifically hybridizing to an mRNA consisting of an base sequence of SEQ ID NO: 5 in the sequence listing.

FIG. 1

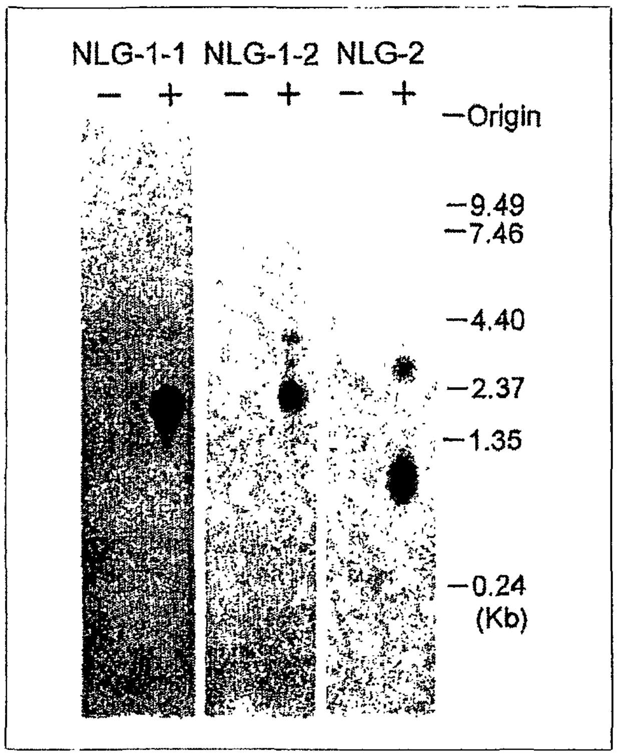
NLG-1-1 NLG-1-2 NLG-2
− + − + − +
Origin
9.49
7.46
4.40
2.37
1.35
0.24
(Kb)

FIG. 2

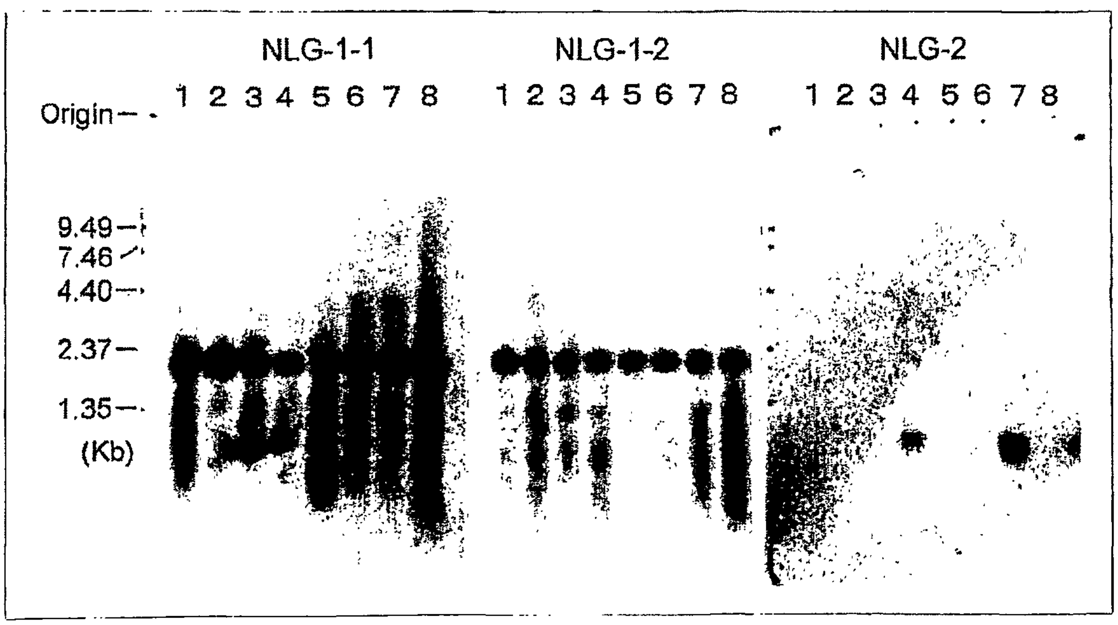
NLG-1-1
NLG-1-2
NLG-2
Origin
1 2 3 4 5 6 7 8
1 2 3 4 5 6 7 8
1 2 3 4 5 6 7 8
9.49
7.46
4.40
2.37
1.35
(Kb)

FIG. 3

B D

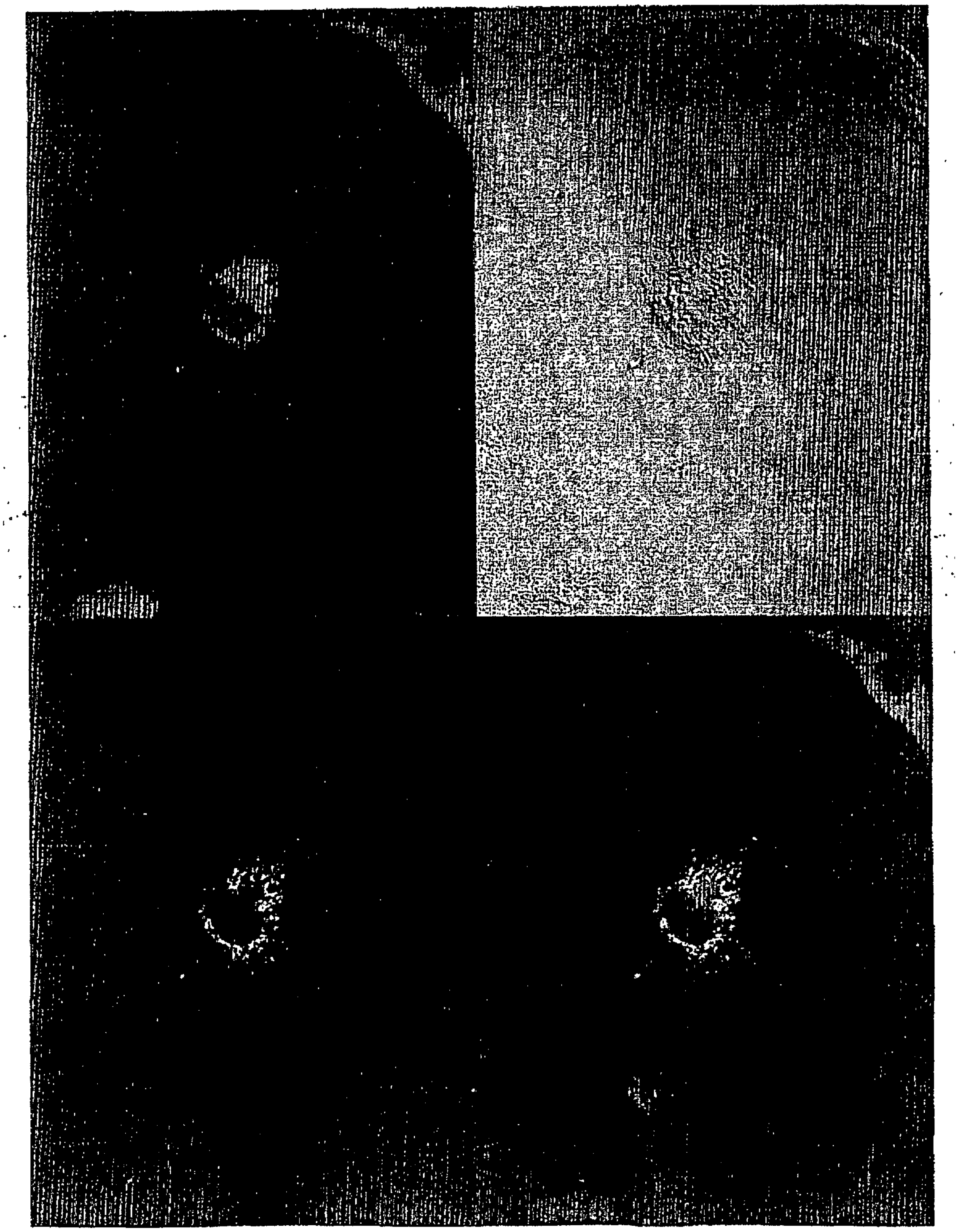

A C

FIG. 4

B D

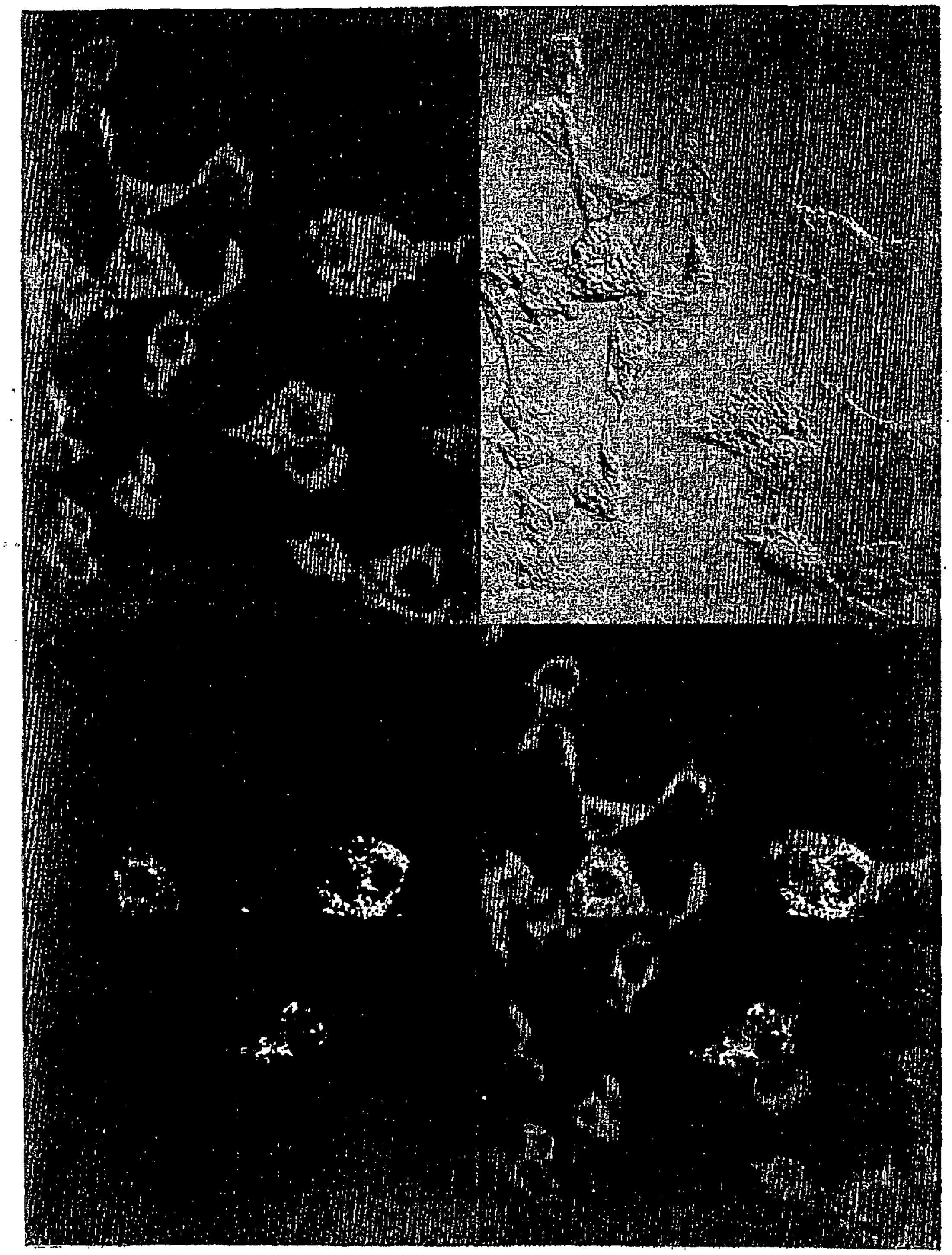

A C

FIG. 5

B

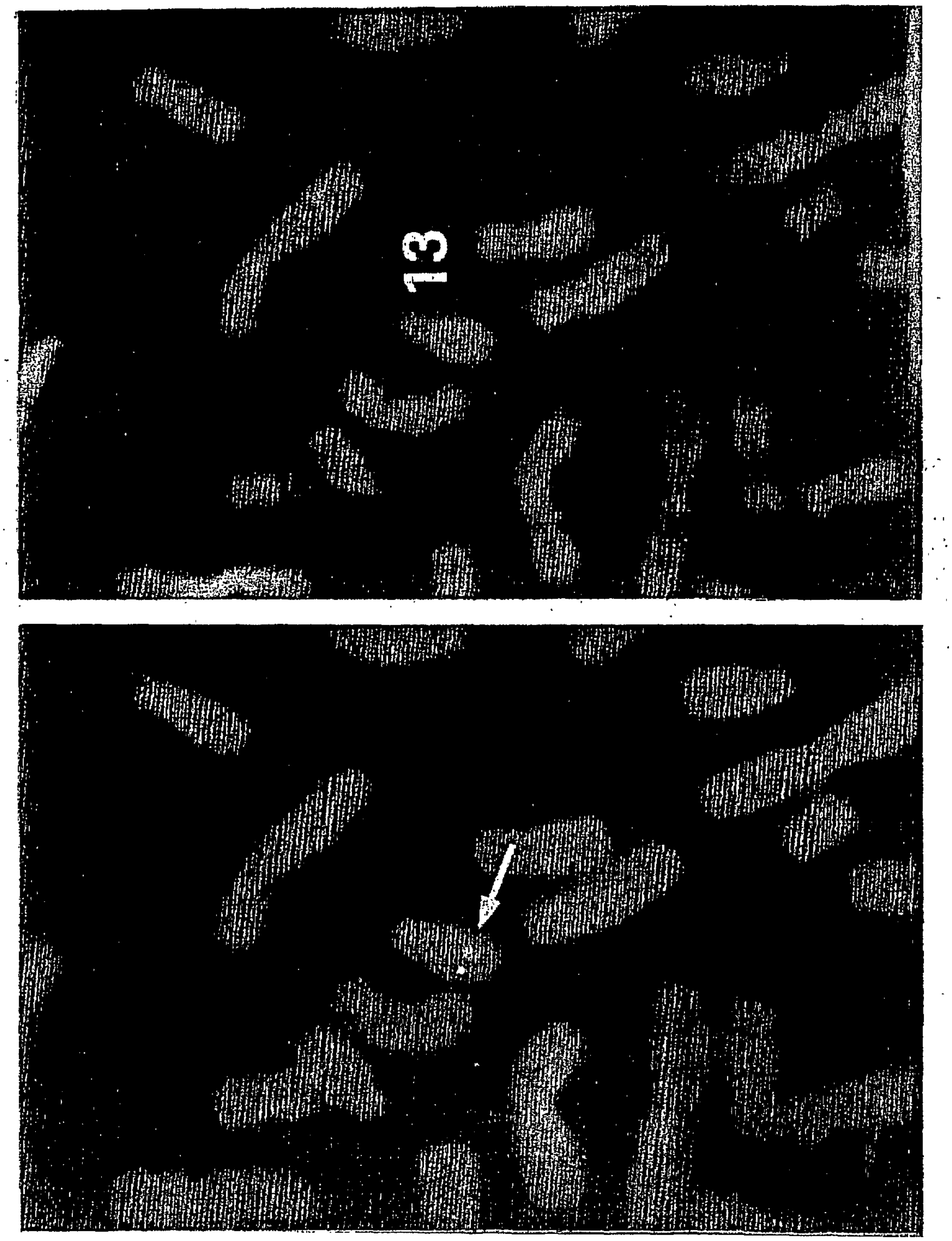


A

FIG. 6

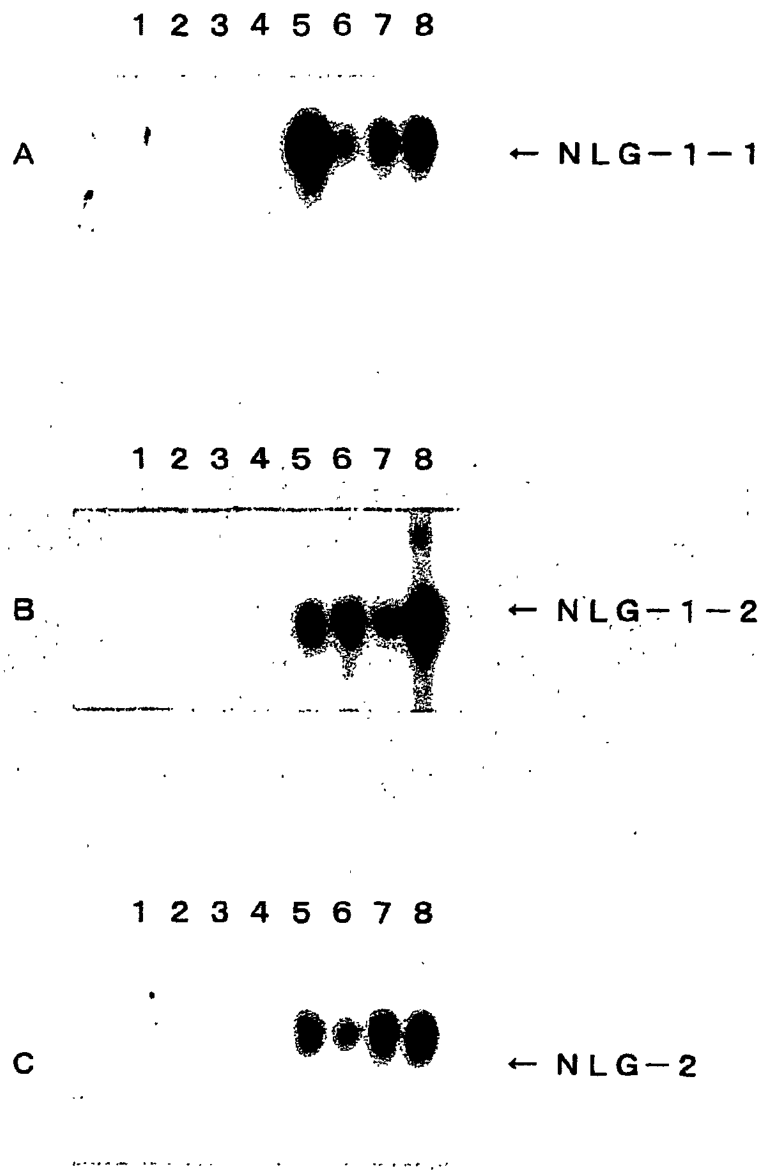

# INTERNATIONAL SEARCH REPORT

International application No.
PCT/JP01/01236

A. CLASSIFICATION OF SUBJECT MATTER
Int.Cl$^7$ C07K 14/47, C12N 15/12, C12N 1/21, C12N 1/19, C12N 5/10, C07K 16/18, C12Q 1/68, G01N 33/53, G01N 33/577 // C12P 21/02

According to International Patent Classification (IPC) or to both national classification and IPC

B. FIELDS SEARCHED

Minimum documentation searched (classification system followed by classification symbols)
Int.Cl$^7$ C07K 14/47, C12N 15/12, C12N 1/21, C12N 1/19, C12N 5/10, C07K 16/18, C12Q 1/68, G01N 33/53, G01N 33/577

Documentation searched other than minimum documentation to the extent that such documents are included in the fields searched

Electronic data base consulted during the international search (name of data base and, where practicable, search terms used)
GenBank/EMBL/DDBJ/GeneSeq,SwissProt/PIR/GeneSeq,REGISTRY(STN),CA(STN),MEDLINE(STN)

C. DOCUMENTS CONSIDERED TO BE RELEVANT

| Category* | Citation of document, with indication, where appropriate, of the relevant passages | Relevant to claim No. |
|---|---|---|
| X | LEE, C. G. et al., "Cloning and analysis of gene regulation of a novel LPS-inducible cDNA", Immunogenetics (1995) Vol.41, No.5, pp.263-270 | 1-18 |
| P,X | WO, 00/57930, A2 (HUMAN GENOME SCIENCES, INC.), 05 October, 2000 (05.10.00) (Family: none) | 1-2,4-6,9-11, 13-15,18 |
| E,X | JP, 2001-069993, A (Japan Science and Technology Corp.), 21 March, 2001 (21.03.01) (Family: none) | 19-27 |
| X | WO, 99/11293, A1 (HUMAN GENOME SCIENCES, INC.), 11 March, 1999 (11.03.99) & AU, 9891304, A1 & EP, 1019091, A1 | 19-24,27 |
| X | US, 5919686, A (INCYTE PHARMACEUTICALS, INC.), 06 July, 1999 (06.07.99) (Family: none) | 19-24,27 |
| P,X | WO, 01/00806, A2 (GENSET), 04 January, 2001 (04.01.01) (Family: none) | 19-24,27 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>09 May, 2001 (09.05.01) | Date of mailing of the international search report<br>22 May, 2001 (22.05.01) |
| Name and mailing address of the ISA/<br>Japanese Patent Office<br><br>Facsimile No. | Authorized officer<br><br>Telephone No. |

Form PCT/ISA/210 (second sheet) (July 1992)

## INTERNATIONAL SEARCH REPORT

International application No.
PCT/JP01/01236

C (Continuation). DOCUMENTS CONSIDERED TO BE RELEVANT

| Category* | Citation of document, with indication, where appropriate, of the relevant passages | Relevant to claim No. |
|---|---|---|
| P,X | WO, 00/55351, A1 (HUMAN GENOME SCIENCES, INC.), 21 September, 2000 (21.09.00) (Family: none) | 19-24,27 |
| P,X | WO, 00/37491, A2 (GENSET), 29 June, 2000 (29.06.00) (Family: none) | 19-24,27 |
| X | WO, 99/06548, A2 (GENSET), 11 February, 1999 (11.02.99) & AU, 9885547, A1 & EP, 1000146, A2 | 19-24,27 |

Form PCT/ISA/210 (continuation of second sheet) (July 1992)

INTERNATIONAL SEARCH REPORT

International application No.
PCT/JP01/01236

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

The inventions as set forth in claims 1 to 27 are divided into three groups of inventions, i.e., a group of inventions relating to proteins containing the amino acid sequence represented by SEQ ID NO:2 (claims 1 to 9), a group of inventions relating to proteins containing the amino acid sequence represented by SEQ ID NO:4 (claims 10 to 18), and a group of inventions relating to proteins containing the amino acid sequence represented by SEQ ID NO:6 (claims 19 to 27). These groups of inventions are not considered as a group of inventions so linked as to form a single general inventive concept.

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☒ No protest accompanied the payment of additional search fees.

Form PCT/ISA/210 (continuation of first sheet (1)) (July 1992)